# EUROPEAN PATENT SPECIFICATION

(11) **EP 1 563 069 B1**
(45) Date of publication and mention of the grant of the patent: **06.06.2012**
(21) Application number: 03780534.8
(22) Date of filing: 24.11.2003
(51) Int. Cl.: C12N 15/11, C12N 15/113, C12N 15/867

(54) **COMPOSITIONS AND SYSTEMS FOR THE REGULATION OF GENES**
ZUSAMMENSETZUNGEN UND SYSTEME ZUR GENREGULIERUNG
COMPOSITIONS ET SYSTEMES DESTINES A LA REGULATION GENIQUE

(30) Priority: 22.11.2002 US 428347 P; 04.06.2003 US 475715 P
(43) Date of publication of application: 17.08.2005
(73) Proprietor: INSTITUT CLAYTON DE LA RECHERCHE, 1205 Genève (CH)
(72) Inventor: TRONO, Didier, CH-1245 Collonge (CH); WIZNEROWICZ, Maciej, Burlington, MA 01803 (US)
(74) Representative: McQueen, Andrew Peter
(86) International application number: PCT/IB2003/006328
(87) International publication number: WO 2004/048583

(56) References cited:
- EP-A- 1 229 134
- WO-A-02/066638
- WO-A-03/022052
- ALLIKIAN MICHAEL J ET AL: "Doxycycline-induced expression of sense and inverted-repeat constructs modulates phosphogluconate mutase (Pgm) gene expression in adult Drosophila melanogaster." GENOME BIOLOGY 2002, vol. 3, no. 5, 15 April 2002 (2002-04-15), page research0021, XP002290537 ISSN: 1465-6914
- SHI H ET AL: "GENETIC INTERFERENCE IN TRYPANOSOMA BRUCEI BY HERITABLE AND INDUCIBLE DOUBLE-STRANDED RNA" RNA, CAMBRIDGE UNIVERSITY PRESS, CAMBRIDGE, GB, vol. 6, no. 7, July 2000 (2000-07), pages 1069-1076, XP008016340 ISSN: 1355-8382
- LAM G ET AL: "INDUCIBLE EXPRESSION OF DOUBLE-STRANDED RNA DIRECTS SPECIFIC GENETIC INTERFERENCE IN DROSOPHILA" CURRENT BIOLOGY, CURRENT SCIENCE,, GB, vol. 10, 24 August 2000 (2000-08-24), pages 957-963, XP001022931 ISSN: 0960-9822
- KAFRI T ET AL: "LENTIVIRAL VECTORS: REGULATED GENE EXPRESSION" MOLECULAR THERAPY, ACADEMIC PRESS, SAN DIEGO, CA,, US, vol. 1, no. 6, 2000, pages 516-521, XP001015529 ISSN: 1525-0016
- HOUDEBINE L-M: "THE METHODS TO GENERATE TRANSGENIC ANIMALS AND TO CONTROL TRANSGENE EXPRESSION" BRAUWELT, NUERNBERG, DE, vol. 98, no. 2/3, 25 September 2002 (2002-09-25), pages 145-160, XP001147895 ISSN: 0168-1656
- MIYAGISHI M ET AL: "U6 PROMOTER-DRIVEN SIRNAS WITH FOUR URIDINE 3' OVERHANGS EFFICIENTLY SUPPRESS TARGETED GENE EXPRESSION IN MAMMALIAN CELLS" NATURE BIOTECHNOLOGY, NATURE PUBLISHING, US, vol. 19, no. 5, May 2002 (2002-05), pages 497-500, XP001153719 ISSN: 1087-0156
- WIZNEROWICZ MACIEJ ET AL: "Conditional suppression of cellular genes: Lentivirus vector-mediated drug-inducible RNA interference." JOURNAL OF VIROLOGY, vol. 77, no. 16, August 2003 (2003-08), pages 8957-8961, XP002290538 ISSN: 0022-538X
- CZAUDERNA FRANK ET AL: "Inducible shRNA expression for application in a prostate cancer mouse model." NUCLEIC ACIDS RESEARCH. 1 NOV 2003, vol. 31, no. 21, 1 November 2003 (2003-11-01), page e127, XP002290539 ISSN: 1362-4962

## Description

### BACKGROUND OF THE INVENTION

### 1. Field of the Invention

The present invention is non-human directed to the fields of molecular biology, gene regulation, and gene therapy and transgenic organisms, More specifically, the present invention relates to methods of controlling gene expression through externally controlled RNA interference systems.

### 2. Description of Related Art

RNA interference (RNAi) is a phenomenon in which an RNA polynucleotide acts through endogenous cellular processes to specifically suppress the expression of a gene whose sequence corresponds to that of the RNA (Brummelkamp *et al.,* 2002; Devroe and Silver, 2002; Barton and Medzhitov, 2002; Xia *et al*., 2002; reviewed in Sharp, 2001). The phenomenon is widespread and apparently evolutionarily conserved (Barton and Medzhitov, 2002; Sui *et al.* 2002). Many studies have now demonstrated that RNAi exists in many organisms and is a naturally occurring cellular process (Sharp, 2001).

The RNAi pathway is not yet completely understood. However, in many systems, small interfering RNA molecules (siRNA) appear to be generated *in vivo* through RNase III endonuclease digestion. The digestion results in molecules that are about 21 to 23 nucleotides (or bases) in length (or size) although molecular size may be as large as 30 bases. These relatively short RNA species then mediate degradation of corresponding RNA messages and transcripts (Sui *et al.* 2002; Sharp 2001). It has been theorized that an RNAi nuclease complex, called the RNA-induced silencing complex (RISC), helps the small dsRNAs recognize complementary mRNAs through base-pairing interactions. Following the siRNA interaction with its substrate, the mRNA is targeted for degradation, perhaps by enzymes that are present in the RISC (Montgomery *et al.,* 1998). These pathways are thought to be useful to the organisms in inhibiting viral infections, transposon jumping, and similar phenomena, and to regulate the expression of endogenous genes (Hutvagner *et al*., 2001; Sharp, 2001; Waterhouse *et al.,* 2001; Zamore 2000).

Although the complete mechanism by which dsRNA suppresses gene expression remains enigmatic, empirical studies demonstrate the effectiveness and importance of RNAi in most organisms.

The ubiquitous presence of RNAi has prompted the development of methods and compositions for turning this natural gene regulation system into a tool for the manipulation of gene expression. One of the most appealing aspects of the use of RNAi for the manipulation of gene expression include its target specificity. RNAi is specific to the sequence of the RNA polynucleotide that mediates the phenomenon. Thus, an RNA polynucleotide sequence designed to correspond sufficiently to the sequence of a gene whose expression is to be suppressed (the target gene) may be introduced into a cell. The presence of the appropriately designed RNA activates the RNAi pathways and result in the suppression or modulation of the target gene.

However, to use RNAi as a means to manipulate gene expression requires that the siRNA be either introduced or expressed with the cell. Current methods of introducing siRNA to the cells in which target gene expression is to be modulated include the direct injection or perfusion of siRNA (or precursor RNA) into the cell, transfection of the cell with an episomal vector (*e.g*. plasmids, adenoviruses, *etc*.), and the permanent introduction into the cell's genome of an expression cassette which expresses the siRNA.

One of the major obstacles to the direct administration of siRNA or precursor RNAs to mammalian cells has been the endogenous antiviral response, which recognizes RNA polynucleotides longer than about 30 nt and degrades them before they may effectively induce RNAi modulation of expression. Elbashir *et al.* (2001) describe the discovery that double stranded RNA polynucleotides 21 bases in length effectively evade this antiviral response and thus may be used to specifically modulate gene expression in mammalian cells.

An alternative means around the antiviral response has been to incorporate an siRNA expression cassette into a vector that after transfection into a cell transcribes the appropriate RNAi inducing RNA species (*see, e.g*., Sui *et al.* 2002; Xia *et al.* 2002; Barton and Medzhitov, 2002). Vectors that may be used include plasmids (Brummelkamp *et al.,* 2002; Sui *et al*., 2002) and virus-derived vectors (*see, e.g.,* Xia et *al.,* 2002; Barton and Medzhitov, 2002; Devroe and Silver, 2002). These presently known systems have been shown to be effective at specific modulation of both exogenous and endogenous genes (*see, e.g*., Sui *et al.* 2002; Xia *et al.* 2002). Viral vectors have the additional advantage that they may result in the stable integration of the siRNA expression construct into the cell's genome, allowing the generation of cell lines with specifically inhibited expression of particular genes. Further, some viral vectors may be utilized to transform cells *in vivo,* thus allowing the direct manipulation of gene expression *in vivo* and in whole organisms.

In the case of stably transfected cells and organisms expressing particular siRNA constructs, one challenge is the avoidance of cellular or organismal toxicity or lowered viability caused by the heretofore constitutive expression of the siRNA products that these constructs provide. Constitutive expression of integrated siRNA in various mammalian systems using a lentiviral vector has been demonstrated by Tiscornia *et al.* (2003). This study has allowed for the generation of cell lines in which expression of specific genes can be reduced and for the generation of knockdown mice with decreased expression of targeted gene products. However, as discussed, constitutive expression of siRNA presents a major obstacle in that during early development this frequently results in early lethality. This abrogates the manipulation of gene expression during development and into the adult stage of an organism. Therefore, there is a need in the art for systems for modulating and controlling gene expression *via* vector borne siRNA wherein the expression of the siRNA molecules themselves may be controlled.

The controlled intracellular transcription of siRNA would be useful for a number of applications, including, for example: controlled production of intracellular or secreted endogenous gene products, such as proteins, from cells, for research of therapeutic purposes; generation of non-human "conditional knockdown" transgenic animals, for instance to create preclinical models of human diseases (*e.g*., diabetes, immunodeficiencies, *etc*.); or to serve as a source of cells/organs for research or therapies, as well as in the agroalimentary industry and for similar objectives in plants; and as a safety device for the clinical application of siRNA, for instance in antiviral therapies or genetic approaches aimed at controlling diseases resulting from the hypersecretion of a hormone, for example.

### SUMMARY OF THE INVENTION

The present invention is directed to systems useful in controlling gene expression through the controlled expression of siRNA. The invention provides externally controllable systems for manipulating the regulation of either endogenous or exogenous genes through controlled RNA interference. The externally controllable systems can be regulated conditionally, in a tissue-specific manner, and/or in a localized manner,

Use of an externally applied agent, such as a drug or one or more other compounds to regulate expression of nucleotide sequences encoding siRNAs, is disclosed.

A system for controlling gene expression is disclosed which comprises a polynucleotide construct comprising a region encoding a siRNA operably linked to an externally controllable promoter, and the construct may be further defined as a vector. A non-limiting example of a vector includes a lentiviral vector, a retroviral vector, an MLV vector, an AAV vector, a plasmid vector or an adenoviral vector. The externally controllable promoter may be a repressible promoter whereby expression of the encoded siRNA can be downregulated by means of an externally applied agent. The expression of the encoded siRNA can be downregulated by means of an externally applied drug.

The externally controllable promoter may be an inducible promoter whereby expression of the encoded siRNA can be upregulated by means of an externally applied agent.

The invention provides a system for controlling gene expression comprising:
(a) a polynucleotide construct comprising a regulatable Pol I or Pol II or Pol III promoter comprising a tetracycline operator (*tetO*) polynucleotide sequence operably linked to at least one polynucleotide encoding siRNAs;
(b) a polynucleotide encoding a drug-controllable repressor fusion protein that comprises the DNA binding domain of the tetracycline repressor (tTR) fused to the KRAB repression domain of human Kox-1 (tTR-KRAB); and
wherein the constructs of (a) and (b) are on one or more vectors.

In particular embodiments, the polynucleotide encoding the fusion protein is operatively linked to an inducible promoter. In additional and preferred embodiments the promoter is a constitutive promoter. In a particular embodiment, the constitutive promoter is the EF-1alpha promoter. In other embodiments the promoter is a tissue-specific promoter.

The various polynucleotides and polynucleotide sequences disclosed may be on or part of a single polynucleotide molecule or they may be located or constitute separate polynucleotide molecules. The different polynucleotides and polynucleotide sequences, if located on the same molecule, may be adjoining, contiguous, next to, or near one another. The arrangement of these sequences will allow for the transcriptional regulation of the siRNA-encoding polynucleotide, and such arrangements can readily be configured by those of ordinary skill in the art. Particular spatial arrangements of relevant sequences are disclosed herein.

In the present invention, control of expression is generated through the use of a particular system comprising both polynucleotide and polypeptide components. In both prokaryotes and eukaryotes, polypeptides having affinity for specific sites on DNA modulate transcriptional expression of genes. Through interaction with DNA at specific sites in genes, certain polypeptides called repressors hinder transcription by, for example, making the DNA inaccessible to RNA polymerase.

DNA-binding proteins have been characterized extensively to determine how these polypeptides actually contact the DNA molecule, for those embodiments concerning repression through direct binding mechanisms, and interact with it to influence gene expression. Some non-limiting examples of these polypeptides include those that comprise the structural motif alpha-helix-tum-alpha-helix (H-T-H). These proteins bind as dimcrs or tetramers to DNA at specific operator sequences that have approximately palindromic sequences. Contacts made by two adjacent alpha helices of each monomer in and around two sites in the major groove of B-form DNA are a major feature in the interface between DNA and these proteins. Proteins that bind in this manner share sequence similarity in the H-T-H region but vary in the extent of similarity in other regions. This group of proteins include, for example, the temperate bacteriophage repressor proteins and Cro proteins, bacterial metabolic repressor proteins such as GalR, LacI, LexA, and TrpR, bacterial activator protein CAP and dual activator/repressor protein AraC, bacterial transposon and plasmid TetR proteins, the yeast mating type regulator proteins MATa1 and MATalpha2 and eukaryotic homeobox proteins.

Other repressors have little or no sequence homology to H-T-H binding proteins and have no H-T-H binding motif. Binding of operators with approximate palindromic sequence symmetry is observed among some proteins of this group, such as Salmonella typhimurium bacteriophage P22 Mnt protein (VERS87a) and E. coli. TyrR repressor protein (DEFE86). Others of this group bind to operator sequences that are partially symmetric (S. typhimurium phage P22 Arc protein, VERS87b; E. coli Fur protein; DEL087; plasmid R6K pi protein, FILU85) or non-symmetric (phage Mu repressor, KRAU86).

A skilled artisan recognizes that a repressor and/or DNA binding domain may comprise a mutation, as compared to wild-type, so long as the mutation docs not deleteriously affect the respective functions of these components, and these mutated components may be utilized in methods and compositions disclosed.

In particular embodiments, a compound may be administered to the cell that modulates the expression of the ETR-KRAB fusion protein. In additional particular embodiments, a compound may be administered to the cell that modulates the binding of the ETR-KRAB fusion protein to the tetO polynucleotide. sequence of the siRNA expression construct. In a particularly preferred embodiment, the compound that may be administered to the cell is doxycycline. Thus, the KRAB repression domain from the human KOX-1 protein is used as a transcriptional repressor (Thiesen *et al*., 1990; Margolin *et al.,* 1994; Pengue *et al.,* 1994; Witzgall *et al.,* 1994). In one embodiment, KAP-1, a KRAB co-repressor, is used with KRAB (Friedman *et al.,* 1996), either as part of the same fusion protein or provided separately.

In the context of the present invention, any vector that can mediate the delivery and genomic integration of the elements (a) and (b) into the target cell, tissue or organism is contemplated to be within the scope of the invention. In particular embodiments, the vector of (b) is a lentiviral vector, an MLV vector, an AAV vector, a plasmid vector or an adenoviral (Adv or Ad) vector. In particularly preferred embodiments, the vector of (b) is a lentiviral vector. In embodiments wherein the vector of (b) is a lentiviral vector, further embodiments include those in which the tetO polynucleotide sequence and the promoter operably linked to the polynucleotide sequence encoding the siRNA and the polynucleotide sequence encoding the siRNA are comprised in the U3 region of the 3' long terminal repeat of the lentiviral vector.

In additional embodiments of the system, the polynucleotide encoding the fusion protein is comprised within a second, separate vector from the vector comprising the constructs of (a). In particular embodiments, the second vector comprising the polynucleotide encoding the fusion protein is a lentiviral vector, a MLV vector, an AAV vector, as plasmid vector or an adenoviral (Adv or Ad) vector. In a preferred embodiment, the second vector comprising the polynucleotide encoding the fusion protein is a lentiviral vector.

In particular embodiments, the regulatable Pol I or Pol II or Pol III promotes of (a) is a polymerase III-dependent promoter such as a U6 or an H1 promoter. In preferred embodiments the polymerase III-dependent promoter of (a) is a U6 promoter. In particularly preferred embodiments, the polynucleotide of (a) encodes siRNA that forms a stem-and-loop structure, or a hairpin, (*i.e*., an sihRNA). The siRNA encoding polynucleotide of (a) is operably linked to tTR-KRAB-responsive promoter. Typically, this tTR-KRAB -responsive promoter comprises a minimal promoter operatively linked to at least one tet operator (*tetO*) sequence. The *tetO* sequence may be obtained, for example, according to Hillen & Wissmann, "Topics in Molecular and Structural Biology," in Protein-Nucleic Acid Interaction, Saeger & Heinemann, eds., Macmillan, London, 1989, Vol. 10, pp. 143-162. Other *tetO* sequences that may be used in the practice of the invention may be obtained from Genbank and/or are disclosed in Waters, S. H. et al. (1983) Nucl. Acids Res. 11:6089-6105; Hillen, W. and Schollmeier, K. (1983) Nucl. Acids Res. 11:525-539; Stuber, D, and Bujard, H. (1981) Proc. Natl. Acad. Sci. USA 78:167-171; Unger, B. et al. (1984) Nucl Acids Res. 12:7693-7703; and Tovar, K. et al. (1988) Mol. Gen.Genet. 215:76-80. One, two, three, four, five, six, seven, eight, nine or ten or more copies of the tet operator sequence may be employed, with a greater number of such sequences allowing an enhanced range of regulation, in some embodiments.

Cells and non-human transgenic animals can be created using any of the systems and constructs described above. These transgenic animals can be controlled to exhibit a knockdown phenotype in a conditional manner.

The invention further provides a transgenic cell comprising the constructs (a) and (b) of claim 1.

It is contemplated that mammalian cells of the invention include non-human undifferentiated cells, such as an oocyte or fertilized cocyte. These cells can be used to create a non-human transgenic animal using techniques that are known to those of skill in the art. Therefore, in some embodiments, the invention includes a non-human transgenic animal capable of exhibiting conditional knockdown of a target gene comprising cells containing mammalian cells described herein. It is specifically contemplated that a founder cell line or animal can be created for use with the tTR-KRAB system described herein. Thus, the invention covers cells and transgenic non-human animals that express tTR-KRAB as a conditional knockdown/expression system (system that conditionally expresses a knockdown molecule or other gene or protein of interest). In particular embodiments, a transgenic non-human animal has one or more cells comprising constructs (a) and (b) of claim 1.

Expression of tTR-KRAB may be conditional, inducible, tissue-specific, constitutive, or locally utilized (such as locally applied). The tTR-KRAB-mediated suppression of a. polymerase III promoter that controls the transcription of an siRNA to create a conditional knockdown animal is contemplated. The use of constructs described above to transfect or infect sex cells, stem cells, or any other undifferentiated cell type that can be used to create non-human transgenic animals is disclosed.

A system in which RNA interference is mediated by an excision, which in turn is controlled by the presence of an enzyme that can excise a nucleic acid fragment is disclosed. It is specifically contemplated that any application discussed with respect to conditional knockdown of a gene may also be implemented with respect to other regulated ways to knockdown a gene, including the use of tissue-specific promoters and/or the use of Cre recombinase. That is, the system may utilize more than one level of regulation, such as, for example, external agent-regulated control of a controllable repressor fusion protein, in addition to regulation at the level of expression of the siRNA being controlled by the controllable repressor fusion protein.

Thus, in some embodiments of the invention, there is a system for regulating expression of an siRNA against a target in a cell comprising an expression construct that can be regulated (that is, "regulatable") and that has an expression cassette containing a nucleic acid segment encoding an siRNA. The nucleic acid segment can be under the control of a promoter, except an excisable fragment is between the segment encoding the siRNA and the promoter and requires excision before the promoter can effect transcription of the siRNA. The fragment contains excision sites, which can be utilized by the appropriate enzyme to excise the fragment; thus, the fragment is an "excisable fragment." Furthermore, the promoter in some embodiments is regulated by a transciption factor, referred to as a "transcription modulator" whose activity can be regulated ("regulatable transcription modulator"). The regulatable transcription modulator is another component of the system, which can be provided in the system as a polynucleotide encoding it. Components of the invention involve various nucleic acid molecules, including segments, fragments, cassettes, and constructs. Such nucleic acid molecules may be RNA or DNA. Moreover, any or all of these molecules may be regulated or manipulated, including by external factors.

These nucleic acid molecules may be at least, at most, or include 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 contiguous nucleotides or basepairs. In some embodiments an expression construct is viral vector, though it may be a nonviral vector such as a plasmid. The viral vector may be an integrating virus, such as a retrovirus or adeno-associated virus. In some embodiments the expression construct is a retrovirus, particularly a lentivirus.

The term "siRNA expression construct" refers to a nucleic acid molecule that can be capable of expressing an siRNA molecule. The term "regulatable siRNA expression construct" means that expression of the siRNA can be regulated, in contrast to constitutive expression in a given cell type or in all cells. The term "expression construct" is understood to include a construct that is a vector. The term "expression cassette" is understood to refer to a nucleic acid region that includes the nucleic acid to be expressed and control regions involved in its expression, including, but not limited to, promoters and enhancers.

The term "regulatable promoter region" means that a promoter region can be controlled so as to modify or alter expression from the promoter. Modification of expression can be positive or negative. Negative modification of expression means that the expression from the promoter may be eliminated, reduced, limited, or restricted. Positive modification of expression means that expression from the promoter may be achieved, increased, augmented, or amplified. "Promoter region" refers to a nucleic acid region that can control and regulate the rate of transcription of a proximate or adjacent gene, cDNA, or other coding region.

In some embodiments of the invention, a nucleic acid molecule including a sequence encoding, one or more polypeptides that serve as a marker. The marker may be used to monitor or assay for whether nucleic acid or a portion thereof has been integrated into another nucleic acid, transfected or introduced into a cell or organism, or excised. It is contemplated that nucleic acid molecules, systems, and organisms of the invention may comprise one, two, three, four, five or more marker polypeptides or nucleic acids encoding marker polypeptides. Different marker polypeptides can be used to monitor different things, as discussed above.

In addition to siRNA-encoding nucleic acid segments, promoters are disclosed, which may contain one or more segments or elements that allow transcription to be modified or regulated. Regulation may be negative or positive. Negative regulation refers to inhibition, reduction, or elimination of transcription from that promoter. Likewise, positive regulation refers to promotion, increase, or induction of transcription from the promoter containing that elements or segment. Promoters are disclosed which contain an element that allows the transcription from the promoter to be repressed or induced. The element may be recognized by a transcription modulator that is regulated either transcriptionally or at the protein level. In the present invention, the element is a *let* operator, which can bind a transcription repressor that contains the DNA binding domain that recognizes this element. In embodiments of the invention, the promoter is a polymerase III-dependent promoter, meaning it requires polymerase III for transcription.

Promoters which may govern spatial and/or temporal expression are disclosed. Thus, it is contemplated that promoters useful with the invention are tissue-specific or developmentally-specific (promoting transcriptions only at certain developmental stages or periods), while in other embodiments, a promoter is inducible, or it is constitutive.

Polypeptides of the invention can be exogenously expressed. The polypeptides may be naturally occurring, wild-type, polymorphic, or mutated. In specific embodiments of the invention, a polypeptide is a fusion or chimeric protein. A chimeric protein is a polypeptide that contains all or a discrete part of two or more polypeptides. A discrete part of a polypeptide refers to an amino acid region that contains an identifiable function or activity. A fusion protein is a type of chimeric protein in which a first polypeptide or part of the first polypeptide is linked end-to-end to a second polypeptide or a part of the second polypeptide. In specific embodiments of the invention, there is a chimeric protein that is a regulatable transcriptional modulator. In some cases, the regulatable transcriptional modulator is a fusion protein with a DNA binding domain from one polypeptide and a transcription repression or activation domain from another. In specific embodiments, the regulatable transcription modulator can be negatively regulated or modified, such as by the binding of a drug. In further embodiments, the regulatable transcription modulator can be negatively regulated by tetracycline or a tetracycline analog, such as doxycycline.

A "tetracycline analog" is any one of a number of compounds that are closely related to tetracycline (Tc) and which bind to the tet repressor with a Kₐ of at least about 10⁶ M⁻¹. Preferably, the tetracycline analog binds with an affinity of about 10⁹ M⁻¹ or greater, e.g. 10⁹ M⁻¹. Examples of such tetracycline analogs include, but are not limited to those disclosed by Hlavka and Boothe, "The Tetracyclines," in Handbook of Experimental Pharmacology 78, R. K. Blackwood et al. (eds.), Springer Verlag, Berlin-New York, 1985; L. A. Mitscher "The Chemistry of the Tetracycline Antibiotics, Medicinal Research 9, Dekker, New York, 1978; Noyee Development Corporation, "Tetracycline Manufacturing Processes," Chemical Process Reviews, Park Ridge, N.J., 2 volumes, 1969; R. C. Evans, "The Technology of the Tetracyclines," Biochemical Reference Series 1, Quadrangle Press, New York, 1968; and H. F. Dowling, "Tetracycline," Antibiotics Monographs, no. 3, Medical Encyclopedia, New York, 1955. Non-limiting examples of tetracycline analogs include anhydrotetracycline, doxycycline, chlorotetracycline, epioxytetracycline, and the like. Certain Tc analogs, such as anhydrotetracycline and epioxytetracycline, have reduced antibiotic activity compared to Tc. Concentrations of the tetracycline or tetracycline analog useful in the present invention are known in the art or are determined by standard means in the art. In specific embodiments, a doxycycline concetration greater than about 10 ng/mL is utilized (Gossen *et al.,* 1995).

The term "transcription modulator" refers to a polypeptide or protein with an activity that directly or indirectly affects transcription, which activity includes, but is not limited to, nucleic acid binding activity, transcriptional activation activity, and/or transcriptional repression activity. Furthermore, the transcription modulator can be "regulatable" in some embodiments of the invention, which means that its activity can be regulated, that is, inhibited, eliminated, reduced, increased, activated, or altered. Regulation may be temporally or spatially limited as well. It is contemplated that an activity of the transcription modulator may be regulated or modified by altering, for example, one or more of the following transcription; translation; mRNA half-life; protein half-life; post-translational modification; localization; nucleic acid or polypeptide binding specificity, rate of dissociation, or affinity; and/or transcriptional activity. Negative regulation or modification refers to a reduction or elimination of activity, while positive regulation or modification refers to an increase or induction of activity. For example, negative modification of a transcriptional repressor may result in alleviation of the repression it is exerting.

Expression of the transcription modulator may be regulated. Its expression may be under the control of a regulatable promoter, such as one that is tissue-specific or inducible. The term "inducible" refers to an activity that can be activated only in response to a specific stimulus, in contrast to a "constitutive" activity. In the context of a "promoter," the term "inducible" means the promoter will promote transcription only under certain conditions, unlike constitutive promoters. A promoter that is inducible is understood to allow for conditional expression. The term "tissue-specific" means that an activity is present only in a specific tissue as opposed being present ubiquitously.

In some embodiments of the invention, regulation is accomplished solely or in part based on the presence of a physical barrier or impediment between a promoter and the nucleic acid segment to be transcribed. In some cases the barrier or impediment may be removed, for example, through excision of a nucleic acid region forming or constituting all or part of the barrier or impediment. Thus, in some embodiments, there is an excisable fragment located between a promoter and a nucleic acid sequence that can be transcribed. More particularly, the fragment prevents the siRNA encoding nucleic acid region from being under the control of the regulatable promoter region. Once it is excised, the siRNA encoding nucleic acid segment is under the control of the regulatable promoter region. An "excisable fragment" refers to a nucleic acid region that may be physically removed due to one or more enzymatic reactions, such as an enzymatic action involving Cre recombinase. In specific aspects of the invention, the excisable fragment has at least two loxP sites flanking it that allow the fragment to be excised by Cre recombinase. The sequence of a loxP site that functions as a recognition site for Cre recombinase is well known to those of skill in the art. Another example of a excision/recombinase system is the well-known flt/frt system, which may be used in the present invention.

Moreover, another level of regulation in the system can be regulation of the expression of an enzyme or polypeptide that controls whether the nucleic acid sequence of interest, *i.e*., the siRNA-encoding nucleic acid, is under the control of a promoter. In other words, the enzyme or polypeptide is conditionally utilizable. The term "conditionally utilizable" means that the enzyme or polypeptide is available only under particular conditions. In some embodiments, Cre-recombinase-encoding polynucleotide is under the control of a tissue-specific or inducible promoter. Expression can be controlled by other means, however, known to those of skill in the art.

Disclosed is a vector or cell that contains, but is not limited to, nucleic acids described above. Any disclosed nucleic acid may be comprised in an expression construct or vector. The expression construct or vector may then be introduced into a cell. In some cases, the cell has i) an expression cassette including the siRNA encoding nucleic acid segment and a regulatable promoter region and ii) a polynucleotide encoding the regulatable transcription modulator. The cell can also include a conditionally utilizable Cre recombinase, which can catalyze excision of an excisable fragment located between the siRNA encoding nucleic acid segment and the regulatable promoter region. It is specifically contemplated the cell may be a prokaryotic cell or a eukaryotic cell, and in some cases, it is a mammalian cell, such as a canine, feline, bovine, ovine, porcine, caprine, rodent, lagomorph, or swine cell. Further, the cell may be a differentiated cell, or in some cases, the cell is a non-human undifferentiated cell, such as an oocyte, fertilized oocyte, or sperm cell. Undifferentiated cells can be employed to create an animal that comprises the regulatable system of the invention.

Disclosed are eukaryotic host cells comprising a DNA molecule encoding an inducible repressor that can repress expression of at least one siRNA of the invention integrated in the host cell and/or comprising a DNA molecule having an siRNA operably linked to an externally controllable promoter, such as one responsive to the inducible repressor, whether it is responsive directly or indirectly. For example, the inducible represser may directly affect sequences nearby the promoter, thereby subsequently affecting the activity of the promoter itself. The host cell can be a mammalian cell (*e.g*., a human cell). Alternatively, the host cell can be a yeast, fungal or insect cell (*e.g*., the inducible repressor or siRNA-encoding DNA can be integrated into a baculovirus gene within an insect cell). A preferred host cell type for homologous recombination is an embryonic stem cell, which can then be used to create a non-human animal currying, for example, t'TR-KRAB-coding sequences integrated at a predetermined location in a chromosome of the animal. A host cell can further contain a siRNAs operably linked to an exemplary tTR-KRAB-responsive transcriptional promoter. The siRNA operably linked to the tTR-KRAB responsive promoter can be integrated into DNA of the host cell either randomly (e.g., by introduction of an exogenous gene) or at a predetermined location (e.g., by targeting an endogenous gene for homologous recombination). The gene linked to the tTR-KRAB-responsive promoter can be introduced into the host cell independently from the DNA encoding the siRNA, for alternatively, a "single hit" targeting vector of the invention can be used to integrate both tTR-KRAB-coding sequences and a tTR-KRAB-responsive promoter into a predetermined location in DNA of the host cell. Expression of the siRNA operably linked to a tTR-KRAB-responsive promoter in a host cell of the invention can be inhibited by contacting the cell with tetracycline or a tetracycline analog.

Disclosed is a transgenic non-human animal comprising a regulatable expression, cassette encoding an siRNA molecule and a polynucleotide encoding the regulatable transcription modulator. Specifically disclosed is a non-human transgenic animal capable of exhibiting conditional knockdown of a target gene in a tissue-specific manner comprising cells containing: i) an siRNA-encoding nucleic acid segment under the control of a regulatable promoter region, wherein the siRNA corresponds to the target gene; ii) the polynucleotide encoding the regulatable transcription modulator, and, iii) a conditionally utilizable Cre recombinase. However, it is specifically contemplated that the tissue-specific aspect may be excluded or that the Cre recombinase level of control may be excluded. Thus, animals are disclosed that do not contain a nucleic acid that is controlled in a tissue-specific manner and/or Cre recombinase and an excisable fragment. The phrase "conditional knockdown of a target gene" means that expression of a target gene is eliminated or substantially eliminated in a condition manner. The disclosure includes include progeny of transgenic animals of the invention, as well as sex cells and other transgenic cells of created transgenic animals.

Other methods are disclosed which include methods of creating or producing a non-human transgenic animal capable of exhibiting conditional knockdown of a target gene using the reagents discussed above. In such cases, a first transgenic animal having conditionally regulated siRNA expression can be mated with a second transgenic non-human animal as the first but of a different gender.

Furthermore, such animals may also be regulated in a conditional and tissue-specific manner comprising: a) obtaining a first transgenic animal having a Cre recombinase-encoding polynucleotide under the control of a tissue-specific promoter; b) obtaining a second transgenic animal having i) an siRNA-encoding nucleic acid segment under the control of a regulatable promoter region, wherein the siRNA corresponds to the target gene; and, ii) the polynucleotide encoding the regulatable polypeptide regulator; and, c) mating opposite sexes of the first and second animals. The second transgenic animal may be obtained by: d) transfecting an undifferentiated non-human mammalian cell with i) a regulatable siRNA-expression construct comprising an siRNA encoding nucleic acid segment and a regulatable promoter region, wherein an excisable fragment is located between the segment and the regulatable promoter region; and ii) a polynucleotide encoding a regulatable polypeptide regulator of the regulatable siRNA-expression construct; e) fertilizing the cell if the cell has a haploid genome; and, f) transplanting the embryo into a non-human female animal, wherein the female animal produces the second transgenic animal. In some cases, a non-human undifferentiated cell is an unfertilized oocyte, a fertilized oocyte, an embryonic stem cell, a cell within a morula or blastocyst. Moreover, methods can also involve culturing the cell prior to transfection and/or transplantation. Further, such methods can involve conventional matings with non-human transgenic animals of the invention.

Disclosed are methods of regulating the expression of a gene in a cell, such as by preparing or providing a region encoding a siRNA operably linked to an externally controllable promoter, wherein the siRNA. encoded by the construct downregulates the expression of the gene; followed by externally regulating the expression of the encoded siRNAs through the externally controllable promoter. The externally controllable promoter may be a repressible promoter whereby expression of the encoded siRNA is downregulated by means of an externally applied agent, such as an externally applied drug. The repressible promote may comprise at least one *tetO* sequence, and additionally the method may further comprise the step of providing a polynucleotide encoding an inducible repressor that can repress the expression of the siRNA. The polynucleotide encoding the repressor may be further defined as a drug-inducible repressor fusion protein that comprises a DNA binding domain and a transcription repression domain, wherein the binding domain of the fusion protein can bind the polynucleotide construct such that the trancription repression domain acts to repress transcription of the siRNA.

Disclosed is a method of studying the function of a gene product in a cell by providing in the cell the following: (i) a polynucleotide construct comprising a region encoding a siRNA operably linked to an externally controllable promoter; wherein the siRNA encoded by the construct downregulated the expression of the gene encoding the gene product; and (ii) a polynucleotide encoding an inducible repressor that can repress the expression of the siRNA; and (b) externally regulating the expression of the encoded siRNA through the externally controllable promoter The externally controllable promoter may be a repressible promoter whereby expression of the encoded siRNA can be downregulated by means of an externally applied agent, such as a drug. Methods and compositions described herein may be utilized for a therapeutic purpose, such as controlling the ability of a cell to be recognized immunologically. In doing.so, the cell is more amenable to cell transplantation through a decrease or inhibition of cell recognizability by the immune system through downregulation of a transplantation antigen, such as a MHC I antigen, for example via down regulation of beta2-microglobulin. Downregulation of beta2-microglobulin may result in downregulation of the whole complex in which it is comprised.

The use of the term "or" in the claims is used to mean "and/or" unless explicitly indicated to refer to alternatives only or the alternative are mutually exclusive, although the disclosure supports a definition that refers to only alternatives and "and/or."

Throughout this application, the term "about" is used to indicate that a value includes the standard deviation of error for the device or method being employed to determine the value.

Following long-standing patent law, the words "a" and "an," when used in conjunction with the word "comprising" in the claims or specification, denotes one or more.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

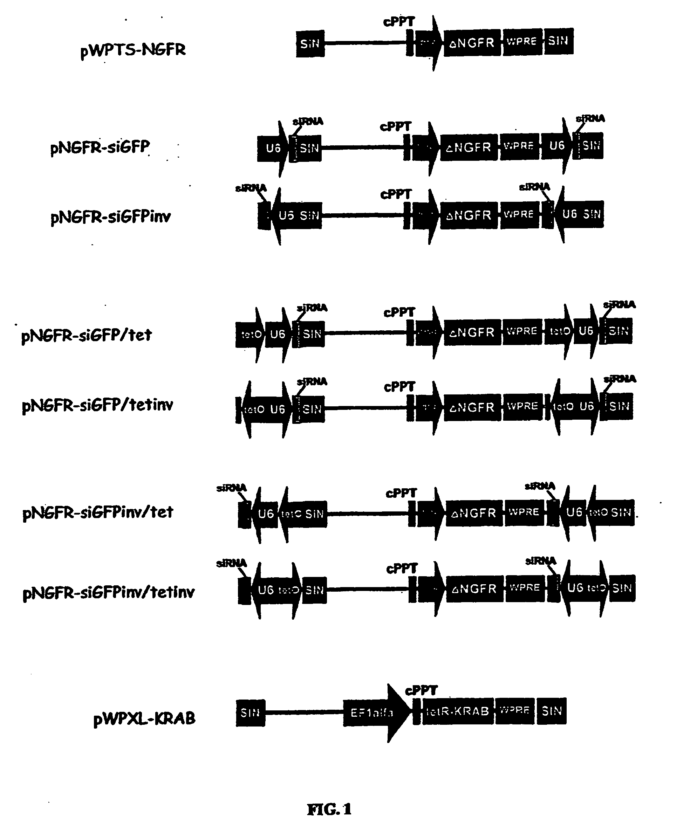

**FIG. 1****.** Lentiviral vectors for regulable shRNA synthesis.

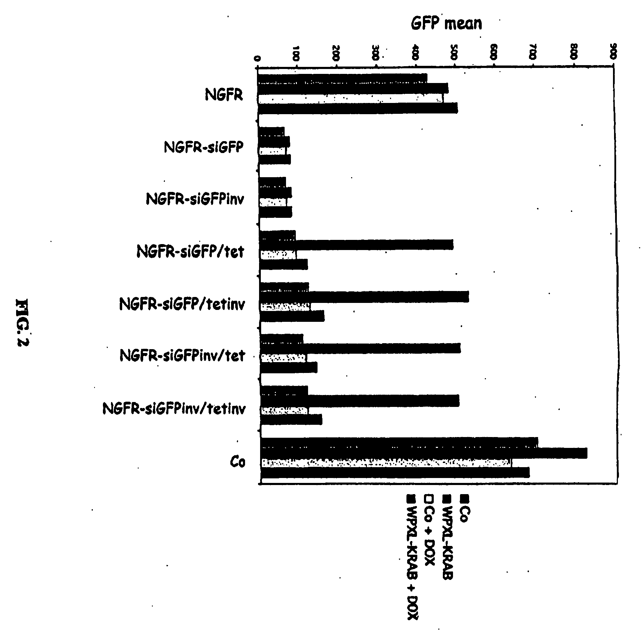

**FIGS. 2****.** Doxycycline-inducible regulation of GFP expression *via* Tet-KRAB-mediated repression of siRNA production. Mean GFP expression is indicated on the vertical axis. The eight bays of the horizontal axis display each construct transfected into the control (Co) cells expressing GFP as indicated. The Co (Control) bay was not transfected with constructs but for the treatments. Four bars within each bay display GFP expression for each construct: Control treatments (Co; first bar of each set of four); WPXL-KRAB treatment, which indicates the co-transfection of the cells with the WPXL-KRAB construct (second bar of four); Doxycycline treatment (DOX; third bar of four); and WPXL-KRAB + DOX, which includes the co-transfection of the cells with the WPXL-KRAB construct and treatment of the cells with Doxycycline.

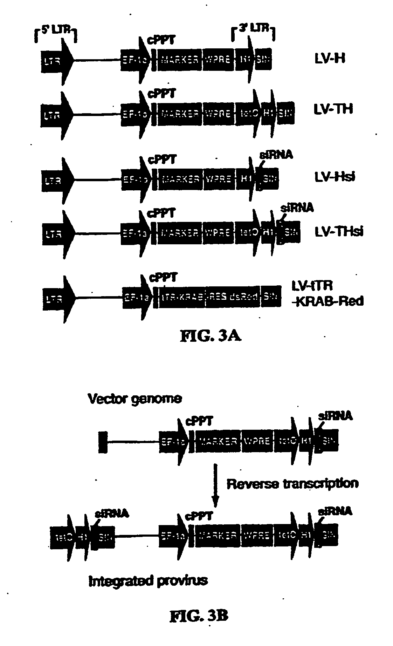

**FIGS. 3A-3B****.** A lentivector-based system for conditional gene suppression with dox-inducible siRNAs. **(****FIG. 3A****)** Schematic drawing of lentiviral vector plasmids used. H1 promoter without (LV-H) or with (pLV-TH) upstream *tetO* sequence, H1-siRNA (LV-Hsi) and *tetO*-H1-siRNA (LV-THsi) cassettes were cloned in the 3' U3 region of pWPXL. All the vectors contain an internal marker cDNA under transcriptional control of the EF-1α promoter. **(****FIG. 3B****)** Double-copy design of siRNA lentivectors. During reverse transcription, the U3 region of the 5' LTR is synthesized using its 3' homologue as a template, which results in a duplication of the siRNA cassette in the provirus integrated in the genome of transduced cells. The constructs in FIG. 3 are also generally described in FIG. 1.

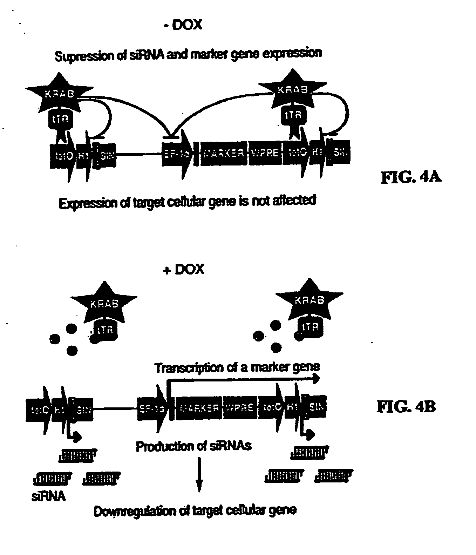

**FIG. 4A-4B****.** Mode of action of the dox-controllable transrepressor. **(****FIG. 4A****)** In the absence of dox, tTR-KRAB binds to *tetO* and suppresses H1-mediated siRNA transcription, thus allowing normal expression of the cellular target gene ("On"). **(****FIG. 4B****)** In the presence of dox, tTR-KRAB cannot bind to *tetO,* hence siRNAs are produced leading to downregulation of their target ("Off"). The internal marker contained in the siRNA vectors provides an "inverse" monitoring device, as it is "on" in the presence of dox and "off" in its absence.

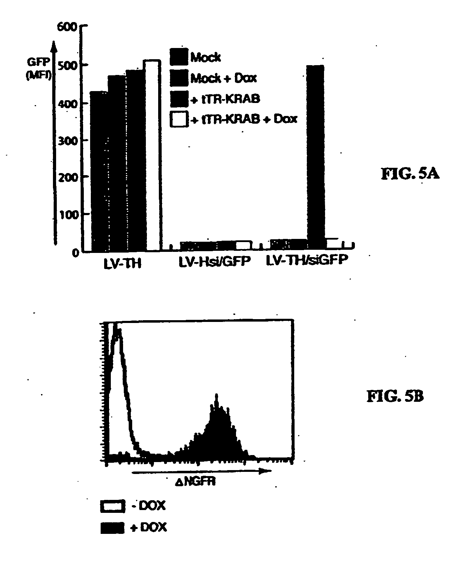

**FIGS. 5A-5B****.** Regulation of GFP expression using dox-inducible siRNA. **(****FIG. 5A****)** Hela cells carrying a single copy of a lentivector expressing GFP from the EF-1α promoter (Hela-GFP) were transduced with a control lentiviral vector (LVTH) or with vectors producing a GFP-specific siRNA in a constitutive (LVHsi) or regulated (LV-THsi) manner, with or without LV-tTR-KRAB (lacking IRES-dsRed cassette) and/or doxycycline as indicated. A truncated form of the nerve growth factor receptor (ΔNGFR) served as an internal reporter in the siRNA vectors. This is similar to the data presented in FIG. 2. **(****FIG. 5B****)** Conditional expression of the internal marker gene. Hela-GFP cells dually transduced with LV-THsi/GFP and LV-tTR-KRAB were maintained in the presence or absence of dox before FACS analysis with a monoclonal Ab specific for the extracellular domain of NGFR.

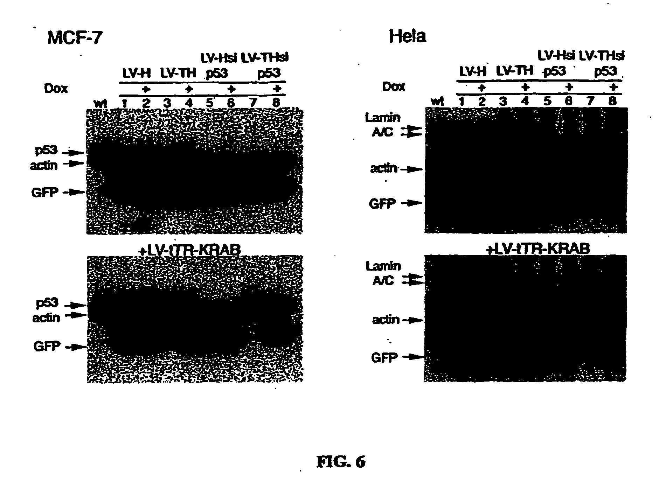

FIG. 6. Regulation of endogenous genes using dox-inducible siRNAs. Left: down modulation of p53. MCF-7 cells were infected with the indicated lentiviral vectors as described in Materials and Methods. Western blot analysis used monoclonal antibodies against p53, GFP or actin (as a control). Right: down modulation of Lamin A/C. Same experiment in Hela cells, using Lamin-specific siRNA vectors and antibodies.

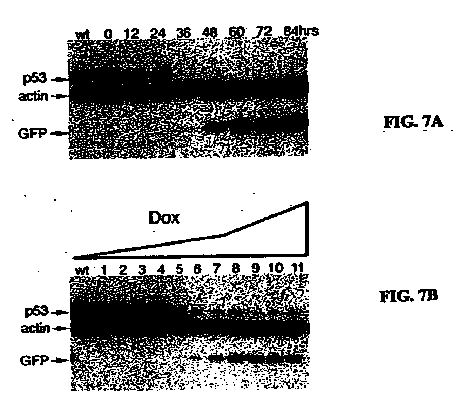

**FIGS. 7A-7B****.** Kinetics and dose-responsiveness of dox-inducible RNA interference. **FIG. 7A****)** MCF-7 cells were cotransduced with LV-THsi/p53 and LV-tTR-KRAB a described in Materials and Methods. Five days later, dox was added at a concentration of 5 µg/ml. Cells were harvested just before dox treatment (lane "0") and then at indicated time points; wt, non-transduced cells. Whole cell extracts were analyzed by Western blot with p53-specific antibodies. **(****FIG. 7B****)** Five days post-transduction as in **(****FIG. 7A****),** cells were placed in medium containing the following concentrations of dox (µg/ml): 0 (lane 1); 0.0005 (lane 2); 0.001 (lane 3); 0.002 (lane 4); 0.004 (lane 5); 0.008 (lane 6); 0.016 (lane 7); 0.063 (lane 8); 0.25 (lane 9); 1 (lane 10); 5 (lane 11); wt, non-transduced cells. Western blot analyses of whole cell extracts were performed after another 5 days.

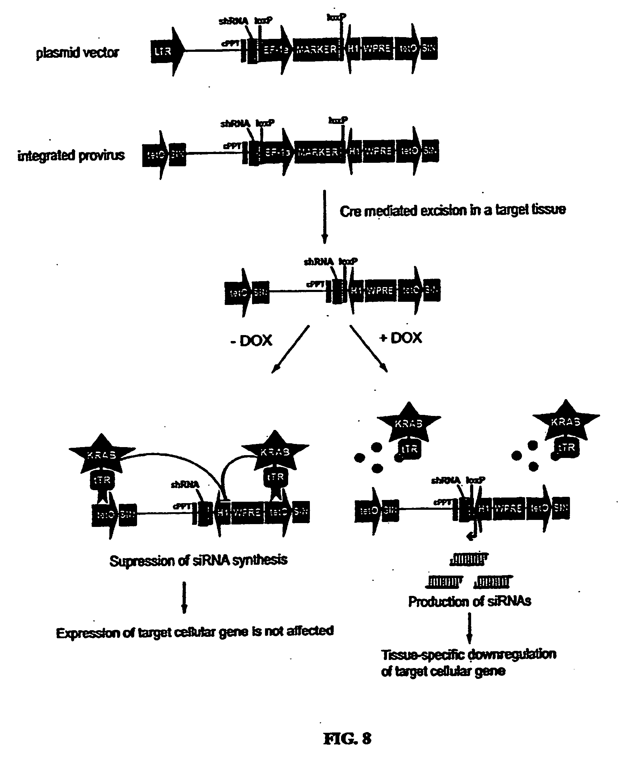

**FIG. 8****.** Cre-mediated activation of HI promoter. Fertilized oocytes retrieved from Transgenic mice expressing Cre in a target tissue are transduced with tet-siRNA lentivectors (as well as LV-tTR-KRAB). The LoxP-flanked EF-1α-MARKER cassette is removed permitting conditional production ofsiRNA in a target tissue.

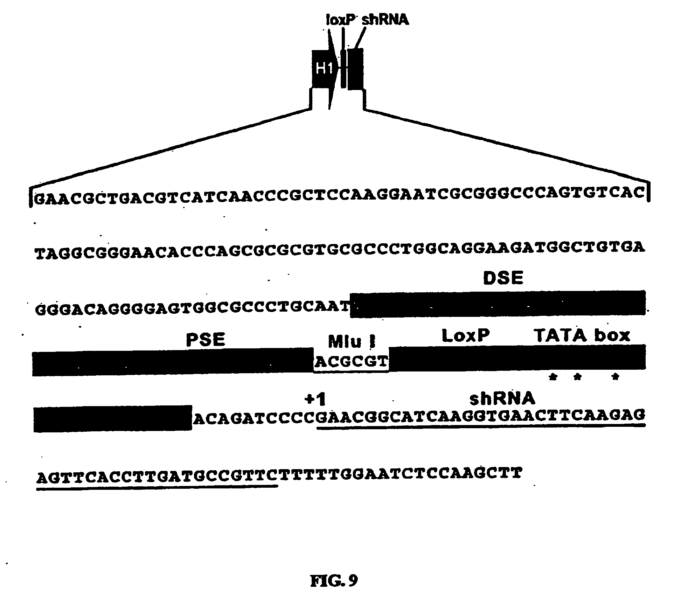

**FIG. 9****.** Sequence of H1 promoter after Cre-mediated excision. LoxP sequence was inserted into H1 promoter between the proximal sequence element (PSE) and transcription start site replacing wt sequences (the wt distance was conserved). LoxP sequence (core element) has been modified (*) to accommodate a TATA box. Sequence of the GFP-specific hairpin was inserted as an example.

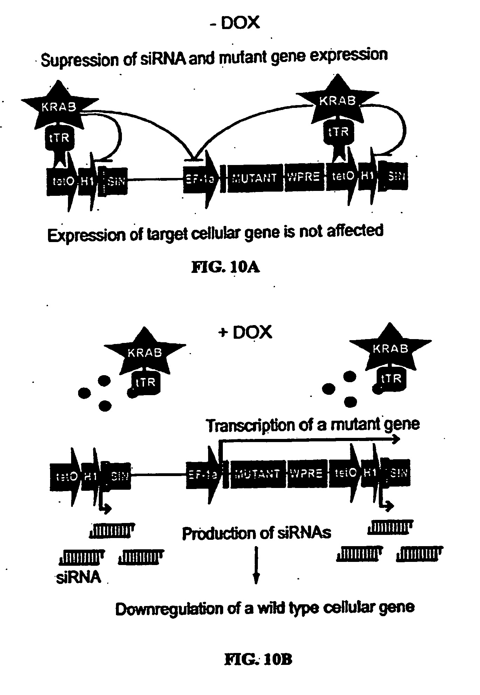

**FIGS. 10A-10B****.** Application of the presented system for analysis of a mutant gene phenotype. The modalities of the presented system allows for mutually exclusive conditional expression of the wild type cellular gene or its mutated form. **(****FIG. 10A****)**. In the absence of drug mutant expression and siRNA, synthesis is suppressed (wt phenotype On). **(****FIG. 10B****)** In the presence of the drug, the wt gene expression is down regulated by an action of siRNA and the mutant form is transcribed.

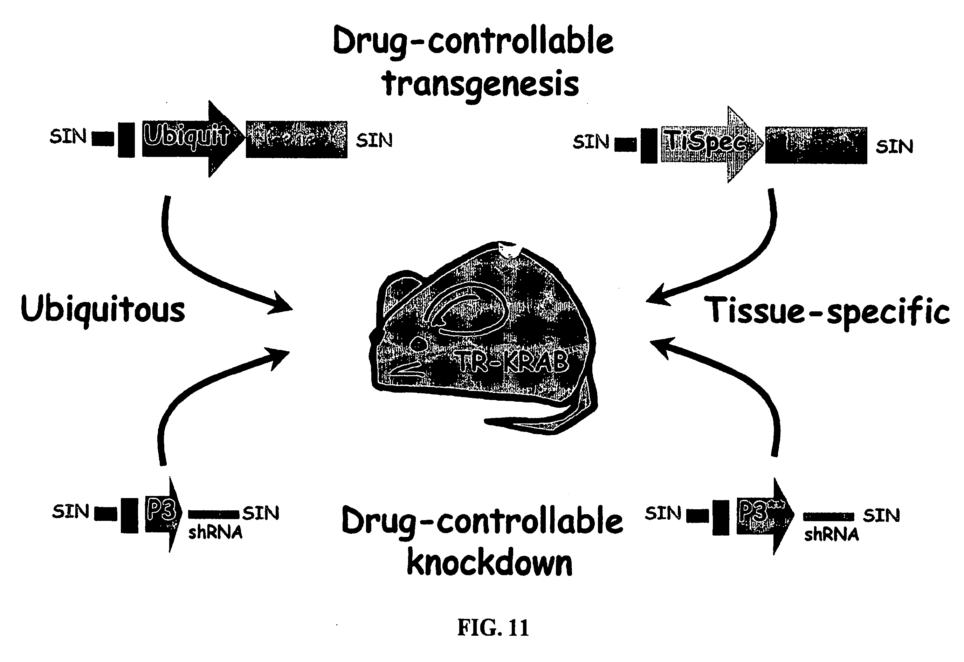

**FIG. 11****.** Illustrative exemplary embodiment of drug-controllable transgenesis and drug-controllable knockdown.

### DETAILED DESCRIPTION OF THE INVENTION

The present invention provides a system for controlling gene expression according to claim 1. . The present invention relates to the application of drug-inducible RNA interference for the development of gene knockdown animals, which optionally may be of lentivector-mediated. The present invention proposes to apply drug inducible RNA interference to generate knockdown animals in which gene function can be modulated externally.

Lentivector-mediated transgenesis has emerged as an efficient and time saving tool to create genetically modified organisms (Lois *et al.,* 2002). Lentivector-delivered RNA interference can also be used to silence gene expression in transgenic mince (Rubinson *et al.,* 2003). The versatility of the mode of delivery suggests very broad uses, as lentiviral vectors can transduce a wide range of targets including stem cells, and can be used for generating transgenic animals from several species.

The presented technology exploits the hollowing system: drug-inducible regulation of polymerase III activity mediated by tetracycline transrepressor (tTR-KRAB); and lentivector mediated transgenesis. The system described herein offers significant advantages over currently available conditional knockout. First, it shortens the time of generating transgenic animal (cost saving, labor saving, time saving) compared to traditional Cre-loxP mediated knock-out strategies. Second, it provides a reversible phenotype of the generated knockdown mice. Third, the target gene can be switched off and back on several times during development or in adulthood (Cre-mediated excision results in irreversible phenotype). Fourth, it allows for the generation of conditional knockdown animals from different animal species besides mice.

### I. RNA interference

The siRNA described herein allows for the modulation and especially the attenuation of target gene expression when such a gene is present and liable to expression within a celt. Modulation of expression can be partial or complete inhibition of gene function, or even the up-regulation of other, secondary target genes or the enhancement of expression of such genes in response to the inhibition of the primary target gene. Attenuation of gene expression may include the partial or complete suppression or inhibition of gene function, transcript processing or translation of the transcript. In the context of RNA interference, modulation of gene expression is thought to proceed through a complex of proteins and RNA, specifically including small, dsRNA that may act as a "guide" RNA. The siRNA therefore is thought to be effective when its nucleotide sequence sufficiently corresponds to at least part of the nucleotide sequence of the target gene. Although the present invention is not limited by this mechanistic hypothesis, it is highly preferred that the sequence of nucleotides in the siRNA be substantially identical to at least a portion of the target gene sequence.

A target gene generally means a polynucleotide comprising a region that encodes a gene product, such as a polypeptide, or a polynucleotide region that regulates replication, transcription or translation or other processes important to the expression of the polypeptide, or a polynucleotide comprising both a region that encodes a polypeptide and a region operably linked thereto that regulates expression. In the context of the invention operably linked refers to the promoter being in a correct functional location and/or orientation in relation to a polynucleotide sequence to control initiation and/or expression of that sequence.

The targeted gene can be chromosomal (genomic) or extrachromosomal. It may be endogenous to the cell, or it may be a foreign gene (a transgene). The foreign gene can be integrated into the host genome, or it may be present on an extrachromosomal genetic construct such as a plasmid or a cosmid. The targeted gene can also be derived from a pathogen, such as a virus, bacterium, fungus or protozoan, which is capable of infecting an organism or cell. Target genes may be viral and pro-viral genes that do not elicit the interferon response, such as retroviral genes. The target gene may be a protein-coding gene or a non-protein coding gene, such as a gene which codes for ribosmal RNAs, splicosomal RNA, tRNAs, *etc*.

Any gene being expressed in a cell can be targeted. Preferably, a target gene is one involved in or associated with the progression of cellular activities important to disease or of particular interest as a research object. Thus, by way of example, the following are classes or possible target genes for the system of the present invention to modulate or attenuate target gene expression: developmental genes (e.g. adhesion molecules, cyclin kinase inhibitors, Wnt family members, Pax family members; Winged helix family members, Hox family members, cytokines/lymphokines and their receptors, growth or differentiation factors and their receptors, neurotransmitters and their receptors); oncogenes (*e.g*. ABLI, BLC1, BCL6, CBFA1, CBL, CSFIR, ERBA, ERBB, EBRB2, ETS1, BTS1, ETV6, FGR, FOX, FYN, HCR, HRAS, JUN, KRAS, LCK, LYN, MDM2, MLL, MYB, MYC, MYCL1, MYCN, NRAS, PIM1, PML, RET, SRC, TAL1, TCL3 and YES), tumor suppresser genes (*e.g*. APC, BRCA1, BRCA2, MADH4, MCC, NF1, NF2, RB1, TP53 and WT1); and enzymes (*e.g*. ACP desaturases and hycroxylases; ADP-glucose pyrophorylases, ATPases, alcohol dehydrogenases, amylases, amyloglucosidases, catalases, cellulases, cyclooxygenases, decarboxylases, dextrinases, esterases, DNA and RNA polymerases, galactosidases, glucanases, glucose oxidases, GTPases, helicases, hemicellulases, integrases, invertases, isomersases, kinases, lactases, lipases, lipoxygenases, lysozymes, pectinesterases, peroxidases, phosphatases, phospholipases, phophorylases, polygalacturonases, proteinases and peptideases, pullanases, recombinases, reverse transcriptases, topoisomerases, xylanases), for example.

The nucleotide sequence of the siRNA is defined by the nucleotide sequence of its target gene. The siRNA contains a nucleotide sequence that is essentially identical to at least a portion of the target gene. Preferably, the siRNA contains a nucleotide sequence that is completely identical to at least a portion of the target gene. Of course, when comparing an RNA sequence to a DNA sequence, an "identical" RNA sequence will contain ribonucleotides where the DNA sequence contains deoxyribonucleotides, and further that the RNA sequence will typically contain a uracil at positions where the DNA sequence contains thymidine.

A siRNA. comprises a double stranded structure, (*e.g*., a hairpin structure or shRNA) the sequence of which is "substantially identical" to at least a portion of the target gene. "Identity," as known in the art, is the relationship between two or more polynucleotide (or polypeptide) sequences, as determined by comparing the sequences. In the art, identity also means the degree of sequence relatedness between polynucleotide sequences, as determined by the match of the order of nuelcotides between such sequences. Identity can be readily calculated. See, for example: Computational Molecular Biology, 1988; Biocomputing: Informatics and Genome Projects, 1993; and the methods disclosed in WO 99/32619, WO 01/68836, WO 00/44914. and WO 01/36646. While a number of methods exist for measuring identity between two nucleotide sequences, the term is well known in the art. Methods for determining identity are typically designed to produce the greatest degree of matching of nucleotide sequence and are also typically embodied in computer programs. Such programs are readily available to those in the relevant art. For example, the GCG program package (Devereux *et al.,* 1984), BLASTP, BLASTN, and FASTA (Altschul *et al*., 1998) and CLUSTAL (Higgins *et al*., 1992; Thompson, *e*t *al.,* 1994).

One of skill in the art will appreciate that two polynucleotides of different lengths may be compared over the entire length of the longer fragment. Alternatively, small regions may be compared. Normally sequences of the same length are compared for a final estimation of their utility in the practice of the present invention. It is preferred that there be 100% sequence identity between the dsRNA for use as siRNA and at least 15 contiguous nucleotides of the targets gene, although a dsRNA having 70%, 75%, 80%, 85%, 90%, or 95% or greater may also be used in the present invention. A siRNA that is essentially identical to a least a portion of the target gene may also be a dsRNA wherein one of the two complementary strands (or, in the case of a self-complementary RNA, one of the two self-complementary portions) is either identical to the sequence of that portion or the target gene or contains one or more insertions, deletions or single point mutations relative to the nucleotide sequence of that portion of the target gene siRNA technology thus has the property of being able to tolerate sequence variations that might be expected to result from genetic mutation, strain polymorphism, or evolutionary divergence.

### II. Controlled expression of siRNA constructs

Controlled expression of siRNA expressing constructs (or any other sequences of interest) may be achieved through a number of systems known to those of skill in the art. As used in the context of the present invention, promoters operate to promote transcription of a polynucleotide. Such a polynucleotide may comprise a sequence encoding an siRNA. An inducible promoter is one whose ability to promote transcription is at least partially responsive to the presence or action of an inducer, which may be a compound or protein that acts to induce the promoter to promote transcription.

### A. Promoters

The term "promoter" as used herein refers to any sequence that regulates the expression of a coding region, such as a gene. Promoters may be constitutive, inducible, repressible, or tissue-specific, for example. A "promoter" is a control sequence that is a region of a polynucleotide sequence at which initiation and rate of transcription are controlled. It may contain genetic elements at which regulatory proteins and molecules may bind such as RNA polymerase and other transcriptions factors. The phrases "operable linked," "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence.

A promoter may be one naturally associated with a gene or sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to promoter that is not normally associated with a nucleic acid sequence in its natural environment. A recombinant or heterologous enhancer refers also to an enhancer not normally associated with a nucleic, acid sequence in its natural environment. Such promoters or enhancers may include promoters or enhancers of other genes, and promoters or enhancers isolated from any other prokaryotic, viral, or eukaryotic cell, and promoters or enhancers not "naturally occurcing," *i.e*., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR™, in connection with the compositions disclosed herein (see U.S. Patent 4,683,202, U.S. Patent 5,928,906). Furthermore, it is contemplated the control sequences that direct transcription and/or expression of sequences within non-nuclear organelles such as mitochondria, chloroplasts, and the like, can be employed as well.

A promoter used in the present invention is an externally controllable promoter, which may be defined as any Pol I or. Pol II. or Pol III promoter (conditional, tissue-specific, regulatable, constitutive, *etc.*) operably linked to at least one tetO polynucleotide sequence bindable by the binding domain of a conditional repressor fusion protein that comprises the DNA binding domain of the tetracycline repressor (tTR) and the KRAB repression domain of human Kox-1, and positioned such that the transcription repression domain acts to repress transcription of a siRNA, or cDNA, or gene.

Inducible promoters are characterized by resulting in additional transcription activity when in the presence of, influenced by, or contacted by the inducer than when not in the presence of, under the influence of, or in contact with the promoter. The inducer may be endogenous, or a normally exogenous compounds or protein that is administered in such a way as to be active in inducing the inducible promoter. Provision of the inducer, *i.e*. a compound or protein, may itself be the result of transcription or expression of a polynucleotide, which itself may be under the control or an inducible or repressible promoter. Examples of inducible promoters include but are not limited to: tetracycline, metallothionine, ecdysone, mammalian viruses (*e.g*, the adenovirus late promoter; and the mouse mammary tumor virus long terminal repeat (MMTV-LTR)) and other steroid-responsive promoters, rapamycin responsive promoters and the like.

The inducible promoters of the present invention are capable of functioning in a eukaryotic host organism. Preferred embodiments include mammalian inducible promoters, although inducible promoters from other organisms as well as synthetic promoters designed to function in a eukaryotic host may be used. The important functional characteristic of the inducible promoters of the present invention is their ultimate inducibility by exposure to an externally applied agent, such as an environmental inducing agent. Appropriate environmental inducing agents include tetracycline. It is important to note that, in certain modes of the invention, the environmental inducing signal can correspond to the removal of any of the above listed agents which are otherwise continuously supplied in the uninduced state. Suitable inducible promoters can be repressed by a transcriptional repressor which itself is rendered inactive by an environmental inducing agent. Thus the inducible promoter can be one which is derepressed by an environmental agent which negatively regulates a transcriptional repressor.

The inducible promoters of the present invention are repressed by a tTR-KRAB repressor fusion protein that is subject to inactivation by the action of tetracycline analog inducing agents. The repressor fusion protein can transcriptionally repress eukaryotic promoters that have been engineered to incorporate appropriate repressor-binding tetracycline operator (tetO) sequences. Thus, where the ETR-KRAB repressor fusion protein is used to control the expression of a eukaryotic promoter that has been engineered to contain a *tetO* operator sequence, treatment of the host cell with tetracycline will cause the dissociation of the repressor from the engineered promoter and allow transcription to occur.

Naturally, it may be important to employ a promoter and/or enhancer that effectively directs the expression of the DNA segment in the cell type, organelle, and organism chosen for expression. Those of skill in the art of molecular biology generally know the use of promoters, enhancers, and cell type combinations for protein expression, for example, see Sambrook *et al.* (1989). The promoters employed may be constitutive, tissue-specific, inducible, and/or useful under the appropriate conditions to direct high level expression of the introduced DNA segment, such as is advantageous in the large-scale production of recombinant proteins and/or peptide. The promoter may be heterologous or endogenous. In certain embodiments, the promoters employed in the present invention are tissue-specific promoters.

Promoters contemplated in the invention include conditional promoters. A conditional promoter is a promoter that is native only under certain conditions. For example, the promoter may be inactive or repressed when a particular agent, such as a chemical compound, is present. When the agent is no longer present, transcription is activated or de-repressed. Examples of conditional promoters may include the promoter Met25 (Kerjan P. *et al.,* 1986), which can be regulated as a function of methionine concentration, or the promoters GAL1 or GAL10 (Johnston and Davis, 1984), which can be regulated as a function of galactose concentration, but are not limited to such,

In preferred embodiments, promoters that are controllable by an external stimulus are utilized in methods and compositions of the present invention. Examples of promoters that are controllable by external stimulus include, for example, the P_{f}. promoter, P_{R} promoter, Pᵣₑ promoter, Pᵣₘ promoter, P'_{R} promoter, T₇ late promoters, trp promoter, tac promoter, lac promoter, gal promoter, ara promoter or recA promoter. In particular embodiments, operator sequences from these promoters are utilize in the invention.

Table 1 lists several elements/promoters that may be employed, in the context of the present invention, to regulate the expression of a gene. This list is not intended to be exhaustive of all the possible elements involved in the promotion of expression but, merely, to be exemplary thereof.

| **TABLE 1** | |
|---|---|
| Promoter and/or Enhancer | |
| Promoter/Enhancer | References |
| Immunoglobulin Heavy Chain | Banerji *et al.,* 1983; Gilles *et al.,* 1983; Grosschedl *et al.,* 1985; Atchinson *et al.,* 1986, 1987; Imler *et al.,* 1987; Weinberger *et al.,* 1984; Kiledjian *et al.,* 1988; Porton et *al*.; 1990 |
| Immunoglobulin Light Chain | Queen *et al.,* 1983; Picard *et al.,* 1984 |
| T-Cell Receptor | Luria *et al.,* 1987; Winoto *et al.,* 1989; Redondo *et al.;* 1990 |
| HLA DQ a and/or DQ β | Sullivan *et al.,* 1987 |
| β-Interferon | Goodboum *et al.,* 1986; Fujita *et al.,* 1987; Goodbourn et *al*., 1988 |
| Interleukin-2 | Greene *et al*., 1989 |
| Interleukin-2 Receptor | Greene *et al.,* 1989; Lin *et al.,* 1990 |
| MHC Class II 5 | Koch *et al.,* 1989 |
| MHC Class II HLA-Dra | Sherman *et al.,* 1989 |
| β-Actin | Kawamoto *et al.,* 1988; Ng *et al.;* 1989 |
| Muscle Creatine Kinase (MCK) | Jaynes *et al.,* 1988; Horlick *et al.,* 1989; Johnson *et al.,* 1989 |
| Prealbumin (Transthyretin) | Costa *et al.,* 1988 |
| Elastase I | Omitz *et al.,* 1987 |
| Metallothionein (MTII) | Karin *et al.,* 1987; Culotta *et al.,* 1989 |
| Collagenase | Pinkert *et al.,* 1987; Angel *et al.,* 1987 |
| Albumin | Pinkert *et al.,* 1987; Tronche *et al.,* 1989, 1990 |
| α-Fetoprotein | Godbout *et al.,* 1988; Campere *et al.,* 1989 |
| t-Globin | Bodine *et al.,* 1987; Perez-Stable *et al.,* 1990 |
| β-Globin | Trudel *et al.,* 1987 |
| c-fos | Cohen *et al.,* 1987 |
| *c-HA-ras* | Triesman, 1986; Deschamps *et al.,* 1985 |
| Insulin | Edlund *et al.,* 1985 |
| Neural Cell Adhesion Molecule (NCAM) | Hirsh *et al.,* 1990 |
| α₁-Antitrypain | Latimer *et al.,* 1990 |
| H2B (TH2B) Histone | Hwang *et al.,* 1990 |
| Mouse and/or Type I Collagen | Ripe *et al.,* 1989 |
| Glucose-Regulated Proteins (GRP94 and GRP78) | Chang *et al.,* 1989 |
| Rat Growth Hormone | Larsen *et al.,* 1986 |
| Human Serum Amyloid A (SAA) | Edbrooke *et al.,* 1989 |
| Troponin I (TN I) | Yutzey *et al.,* 1989 |
| Platelet-Derived Growth Factor (PDGF) | Pech *et al.,* 1989 |
| Duchenne Muscular Dystrophy | Klamut *et al.,* 1990 |
| SV40 | Banerji *et al.,* 1981; Moreau *et al.,* 1981; Sleigh *et al.,* 1985; Firak *et al.,* 1986; Herr *et al.,* 1986; Imbra *et al.,* 1986; Kadesch *et al.,* 1986; Wang *et al.,* 1986; Ondek et *al.,* 1987; Kuhl *et al.,* 1987; Schaffner *et al.,* 1988 |
| Polyoma | Swartzendruber *et al.,* 1975; Vasseur *et al.,* 1980; Katinka *et al.,* 1980, 1981; Tyndell *et al.,* 1981; Dandolo *et al.,* 1983; de Villiers *et al.,* 1984; Hen *et al.,* 1986; Satake *et al.,* 1988; Campbell *et al.,* 1988 |
| Retroviruses | Kriegler *et al.,* 1982, 1983; Levinson *et al.,* 1982; Kriegler *et al.,* 1983, 1984a, b, 1988; Bosze *et al.,* 1986; Miksicek *et al.,* 1986; Celander *et al.,* 1987; Thiesen *et al.,* 1988; Celander *et al.,* 1988; Chol *et al.,* 1988; Reisman *et al.,* 1989 |
| Papilloma Virus | Campo *et al.,* 1983; Lusky *et al.,* 1983; Spandidos and Wilkie, 1983; Spalholz *et al.,* 1985; Lusky *et al.,* 1986; Cripe *et al.,* 1987; Gloss *et al.,* 1987; Hirochika *et al.,* 1987; Stephens *et al.,* 1987 |
| Hepatitis B Virus | Bulla *et al.,* 1986; Jameel *et al.,* 1986; Shaul *et al.,* 1987; Spandau *et al.,* 1988; Vannice *et al.,* 1988 |
| Human Immunodeficiency Virus | Muesing *et al.,* 1987; Hauber *et al.,* 1988; Jakobovits *et al.,* 1988; Feng *et al.,* 1988; Takebe *et al.,* 1988; Rosen et *al.,* 1988; Berkhout *et al.,* 1989; Laspia *et al.,* 1989; Sharp *et al.,* 1989; Braddock *et al.,* 1989 |
| Cytomegalovirus (CMV) | Weber *et al.,* 1984; Boshart *et al.,* 1985; Foecking *et al.,* 1986 |
| Gibbon Ape Leukemia Virus | Holbrook *et al.,* 1987; Quinn *et al.,* 1989 |

Table 2 provides examples of inducible elements, which are regions of a nucleotide sequence that can be activated in response to a specific stimulus. This list is not intended to be exhaustive of all the possible elements involved in the promotion of expression but, merely, to be exemplary thereof.

| **TABLE 2** | | |
|---|---|---|
| Inducible Elements | | |
| Element | Inducer | References |
| MT II | Phorbol Ester (TFA) Heavy metals | Palmiter *et al.,* 1982; Haslinger *et al.,* 1985; Searle *et al.,* 1985; Stuart *et al.,* 1985; Imagawa et *al.,* 1987, Karin *et al.,* 1987; Angel *et al.,* 1987b; McNeall *et al.*, 1989 |
| MMTV (mouse mammary tumor virus) | Glucocorticoids | Huang *et al.,* 1981; Lee *et al.,* 1981; Majors *et al.,* 1983; Chandler *et al.,* 1983; Lee *et al*., 1984; Ponta *et al.,* 1985; Sakai *et al.,* 1988 |
| β-Interferon | poly(rI)x poly(rc) | Tavernier *et al.,* 1983 |
| Adenovirus 5 E2 | ElA | Imperiale *et al.,* 1984 |
| Collagenase | Phorbol Ester (TPA) | Angel *et al.,* 1987a |
| Stromelysin | Phorbol Ester (TPA) | Angel *et al.,* 1987b |
| SV40 | Phorbol Ester (TPA) | Angel *et al.,* 1987b |
| Murine MX Gene | Interferon, Newcastle Disease Virus | Hug *et al.,* 1988 |
| GRP78 Gene | A23187 | Resendez *et al.,* 1988 |
| α-2-Macroglobulin | IL-6 | Kunz *et al.,* 1989 |
| Vimentin | Serum | Rittling *et al.,* 1989 |
| MHC Class I Gene H-2κb | Interferon | Blanar *et al.,* 1989 |
| HSP70 | ElA, SV40 Large T Antigen | Taylor *et al.,* 1989, 1990a, 1990b |
| Proliferin | Phorbol Ester-TPA | Mordacq *et al.,* 1989 |
| Tumor Necrosis Factor | PMA | Hensel *et al.,* 1989 |
| Thyroid Stimulating Hormone α Gene | Thyroid Hormone | Chatterjee *et al.,* 1989 |

A repressible promoter is one whose ability to promote transcription is at least partially responsive to the presence or action of a repressor, which is a compound or protein that acts to repress the promoter and so reduce, inhibit, or repress transcription of the polynucleotide under the influence of the promoter. Repressible promoters are characterized by resulting in lower levels of transcription activity when in the presence of, influenced by, or contacted by the repressor than when not in the presence of, under the influence of, or in contact with the promoter. The repressor may be endogenous, or a normally exogenous compound or protein that is administered in such a way as to be active in repressing the repressible promoter. Provision of the repressor, i.e. a compound or protein, may itself be the result of transcription or expression of a polynucleotide, which itself may be under the control or an inducible or repressible promoter.

In specific embodiments, the polynucleotide molecule coding for the conditional repressor fusion protein and/or the polynucleotide encoding the siRNA further comprises an operably linked promoter. The promotor may be an inducible promoter or a constitutive promoter, in some embodiments. Examples of such promoters include the human cytomegalovirus promoter IE as taught by Boshart *et al*., (1985), ubiquitously expressing promoters such as HSV-Tk (McKnight *et al.,* (1984) and β-actin promoters (*e.g.* the human β-action promoter as described by Ng *et al.,* (1985)), as well as promoters in combination with control regions allowing integration site independent expression of the transgene (Grosveld *et al.,* (1987)), as well as tissue specific promoters such as albumin (liver specific, Pinkert *et al.,* (1987)), lymphoid specific promoters (Calame and Eaton, 1988), in particular promoters of T-cell receptors (Winoto and Baltimore, (1989)) and immunoglobulins; Banerji *et al.,* (1983); Queen and Baltimore, 1983), neuron specific promoters (e.g. the neurofilament promoter; Byrne and Ruddle, 1989), pancreas specific promoters (Edlund *et al.,* (1985)) or mammary gland specific promoters (milk whey promoter, U.S. Pat. No. 4,873,316 and European Application Publication No. 264,166) as well as developmentally regulated promoters such as the murine hox promoters (Kessel and Cruss, Science 249:374-379 (1990)) or the α-fetoprotein promoter (Campes and Tilghman, Genes Dev. 3:537-546 (1989)). Preferably, the promoter is constitutive in the respective cell types.

The identity of tissue-specific promoters or elements, as well as assays to characterize their activity, is well known to those of skill in the art. Examples of such regions include the human LIMK2 gene (Nomoto *et al.* 1999), the somatostatin receptor 2 gene (Kraus *et al.,* 1998), murine epididymal retinoic acid-binding gene (Lareyre *et al.,* 1999), human CD4 (Zhao-Emonet *et al.,* 1998), mouse alpha2 (XI) collagen (Tsumaki, et *al.,* 1998), D1A dopamine receptor gene (Lee, *et al.,* 1997), insulin-like growth factor II (Wu *et al.,* 1997), human platelet endothelial cell adhesion molecule-1 (Almendro *et al.,* 1996), and the SM22α promoter. Tissue-specific promoters and/or regulatory elements will be useful in certain embodiments. Other examples of such tissue-specific promoters that may be used with the expression vectors of the invention include promoters from the liver fatty acid binding (FAB) protein gene, specific for colon epithelial cells; the insulin gene, specific for pancreatic cells; the transphyretin, α1-antitrypsin, plasminogen activator inhibitor type 1 (PAI-1), apolipoprotein AI and LDL receptor genes, specific for liver cells; the myelin basic protein (MBP) gene, specific for oligodendrocytes; the glial fibrillary acidic protein (GFAP) gene, specific for glial cells; OPSIN, specific for targeting to the eye; and the neural-specific enolase (NSE) promoter that is specific for nerve cells.

In certain aspects to the invention, the tissue-specific nature of control is utilized at the level of control of a target sequence, such as by the level of control of the expression of an siRNA, or at the level of another component of regulation, such as expression of an enzyme that directly or indirectly regulates expression of the transgene (siRNA). This is as opposed to the level of expression of the conditional repressor fusion protein. However, in alternative embodiments the expression of the conditional repressor fusion protein is tissue-specific instead of or in addition to the tissue-specific expression of the siRNA or enzyme.

Also contemplated as useful in the present invention are the dectin-1 and dectin-2 promoters. Additional viral promoters, cellular promoters/enhancers and inducible promoters/enhancers that could be used in combination with the present invention are listed in Tables 1 and 2. Additionally any promoter/enhancer combination (as per the Eukaryotic Promoter Data Base EPDB) could also be used to drive expression of structural genes encoding oligosaccharide processing enzymes, protein folding accessory proteins, selectable marker proteins or a heterologous protein of interest. Alternatively, a tissue-specific promoter for gene therapy (Table 3 and Table 4), such as cancer gene therapy, may be employed in the present invention.

**TABLE 3: Candidate Tissue-Specific Promoters for Gene Therapy**

| **Tissue-specific promoter** | **Cancers in which promoter is active** | **Normal cells in which promoter is active** |
|---|---|---|
| Carcinoembryonic antigen (CEA)* | Most colorectal carcinomas; 50% of lung carcinomas; 40-50% of gastric carcinomas; most pancreatic carcinomas; many breast carcinomas | Colonic mucosa; gastric mucosa; lung epithelia; eccrine sweat glands; cells in testes |
| Prostate-specific antigen (PSA) | Most prostate carcinomas | Prostate epithelium |
| Vasoactive intestinal peptide (VIP) | Majority of non-small cell lung cancers | Neurons; lymphocytes; mast cells; eosinophils |
| Surfactant protein A (SP-A) | Many lung adenocarcinomas cells | Type II pneumocytes; Clara |
| Human achaete-scute homolog (hASH) | Most small cell lung cancers | Neuroendocrine cells in lung |
| Mucin-1 (MUC1)** | Most adenocarcinomas (originating from any tissue) | Glandular epithelial cells in breast and in respiratory, gastrointestinal, and genitourinary tracts |
| Alpha-fetoprotein | Most hepatocellular carcinomas; possibly many testicular cancers | Hepatocytes (under certain conditions); testis |
| Albumin | Most hepatocellular carcinomas | Hepatocytes |
| Tyrosinase | Most melanomas | Melanocytes; astrocytes; Schwann cells; some neurons |
| Tyrosine-binding protein (TRP) | Most melanomas | Melanocytes; astrocytes, Schwann cells; some neurons |
| Keratin 14 | Presumably many squamous cell carcinomas (*e.g*.: Head and neck cancers) | Keratinocytes |
| EBV LD-2 | Many squamous cell carcinomas of head and neck | Keratinocytes of upper digestive Keratinocytes of upper digestive tract |
| Glial fibrillary acidic protein (GFAP) | Many astrocytomas | Astrocytes |
| Myelin basic protein (MBP) | Many gliomas | Oligodendrocytes |
| Testis-specific angiotensin-converting enzyme (Testis-specific ACE) | Possibly many testicular cancers | Spermatazoa |
| Osteocalcin | Possibly many osteosarcomas | Osteoblasts |

**TABLE 4: Candidate Promoters for Use with a Tissue-Specific Gene**

| **Promoter** | **Cancers in which Promoter is active** | **Normal cells in which Promoter is active** |
|---|---|---|
| E2F-regulated promoter | Almost all cancers | Proliferating cells |
| HLA-G | Many colorectal carcinomas; many melanomas; possibly many other cancers | Lymphocytes; monocytes; spermatocytes; trophoblast |
| FasL | Most melanomas; many pancreatic carcinomas; most astrocytomas possibly many other cancers | Activated leukocytes: neurons; endothelial cells; keratinocytes; cells in immunoprivileged tissues; some cells in lungs, ovaries, liver, and prostate |
| Myc-regulated promoter | Most lung carcinomas (both small cell and non-small cell); most colorectal carcinomas | Proliferating cells (only some cell-types): mammary epithelial cells (including non-proliferating) |
| MAGE-1 | Many melanomas; some non-small cell lung carcinomas; some breast carcinomas | Testis |
| VEGF | 70% of all cancers (constitutive overexpression in many cancers) | Cells at sites of neovascularization (but unlike in tumors, expression is transient, less strong, and never constitutive) |
| BFGF | Presumably many different cancers, since bFGF expression is induced by ischemic conditions | Cells at sites of ischemia (but unlike tumors, expression is transient, less strong, and never constitutive) |
| COX-2 | Most colorectal carcinomas; many lung carcinomas; possibly many other cancers | Cells at sites of inflammation |
| IL-10 | Most colorectal carcinomas; many lung carcinomas; many squamous cell carcinomas of head and neck; possibly many other cancers | Leukocytes |
| GRP78/BiP | Presumably many different cancers, since GRP7S expression is induced by tumor-specific conditions | Cells at sites of ishemia |
| CarG elements from Eg-1 | Induced by ionization radiation, so conceivably most tumors upon irradiation | Cells exposed to ionizing radiation; leukocytes |

### B. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites (IRES) elements are used to create multigene, or polycistronic, messages. IRES elements are able to bypass the ribosome scanning model of 5'- methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent 5,925,565 and 5,935,819).

### C. Multiple Cloning Sites

Vectors can include a multiple cloning site (MCS), which is a nucleic acid region that contains multiple restriction enzyme sites, any of which can be used in conjunction with standard recombinant technology to digest the vector. (See Carbonelli *et al.,* 1999, and Cocea, 1997.) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### D. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression. (See Chandler *et al.,* 1997 )

### E. Termination Signals

The vectors or constructs of the present invention will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and/or to minimize read through from the cassette into other sequences.

Terminators contemplated for use in the invention include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### F. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Polyadenylation may increase the stability of the transcript or may facilitate cytoplasmic transport.

### G. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### H. Selectable and Screenable Markers

In certain embodiments of the invention, cells containing a polynucleotide construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (tk) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

By way of example, Mermad *et al.* disclose in U.S. Patent 6,340,741 that controlled expression of target genes within eukaryotic systems is widely used in biological and medical research, as well as biotechnology and somatic gene therapy. Regulated gene expression has been achieved by the use of heterologous or artificial (chimerical) transcription factors responding to an exogenously added inducer drug which acts as a bona fide ligand. Typically, these transcription factors recognize cognate regulatory elements in the promoter of the target gene and the ligand regulates the interaction of the factor with the DNA or the interaction of the DNA-bound factor with a transcriptional activation domain.

The administration or removal of the ligand results in a switch between the on or off states of the transcription or activity of the target gene. Several small molecule ligands have been shown to mediate regulated gene expressions, either in tissue culture cells and/or in transgenic animal models. These include the FK1012 and rapamycin immunosupressive drugs (Spencer *et al.,* 1993; Magari *et al.,* 1997), the progesterone antagonist mifepristone (RU486) (Wang, 1994; Wang *et al.,* 1997), the tetracycline antibiotic derivatives (Gossen and Bujard, 1992; Gossen *et al.,* 1995; Kistner *et al.,* 1996), and the insect steroid hormone ecdysone (No *et al.,* 1996).

By way of further example, Yao discloses in U.S. Patent 6,444,871 that in the case of prokaryotic elements associated with the tetracycline resistance (tet) operon, systems have been developed in which the tet repressor protein is fused with polypeptides known to modulate transcription in mammalian cells. The fusion protein has then been directed to specific sites by the positioning of the tet operator sequence. For example, the tet repressor has been fused to a transactivator (VP16) and targeted to a tet operator sequence positioned upstream from the promoter of a selected gene (Gussen *et al.,* 1992; Kim *et al.,* 1995; Hennighausen *et al.,* 1995). The tet repressor portion of the fusion protein binds to the operator thereby targeting the VP16 activator to the specific site where the induction of transcription is desired. An alternative approach has been to fuse the tet repressor to the KRAB repressor domain and target this protein to an operator placed several hundred base pairs upstream of a gene. Using this system, it has been found that the chimeric protein, but not the tet repressor alone, is capable of producing a 10 to 15-fold suppression of CMV-regulated gene expression (Deuschle *et al.,* 1995).

### I. Regulatory Elements and Systems

In some instances of gene regulation and its modification, it is desirable to introduce regulatory elements from evolutionarily distant species such as E. coli into higher eukaryotic cells with the anticipation that effectors that modulate such regulatory circuits will be inert to eukaryotic cellular physiology and, consequently, will not elicit undesirable pleiotropic effects in eukaryotic cells. For example, the Lac repressor (lacR)/operator/inducer system of E. coli functions in eukaryotic cells and has been used to regulate gene expression by three different approaches: (1) prevention of transcription initiation by properly placed lac operators at promoter sites (Hu and Davidson, 1987; Brown *et al.,* 1987; Figge *et al.,* 1988; Fuerst *et al.,* 1989; Deuschle *et al.,* 1989; (2) blockage of transcribing RNA polymerase II during elongation by a LacR/operator complex (Deuschle *et al.* (1990); and (3) activation of a promoter responsive to a fusion between LacR and the activation domain of herpes simples virus (HSV) virion protein 16 (VP16) (Labow *et al.,* 1990; Baim *et al.,* 1991).

In one version of the Lac system, expression of lac operator-linked sequences is constitutively activated by a LacR-VP16 fusion protein and is turned off in the presence of isopropyl-β-D-thiogalactopyranoside (IPTG) (Labow *et al.* (1990), cited supra). In another version of the system, a lacR-VP16 variant is used which binds to lac operators in the presence of IPTG, which can be enhanced by increasing the temperature of the cells (Baim *et al.* (1991), cited supra). Thus, in some embodiments of the present invention, components of the Lac system are utilized. For example, a lac operator may be operably linked to a siRNA-encoding polynucleotide, and its expression may be externally controlled with, for example, IPTG-inducible fusion proteins comprising the lac repressor.

Components of the tetracycline (Tc) resistance system of E. coli have also been found to function in eukaryotic cells and have been used to regulate gene expression. For example, the Tet repressor (TetR), which binds to tet operator sequences in the absence of tetracycline and represses gene transcription, has been expressed in plant cells at sufficiently high concentrations to repress transcription from a promoter containing tet operator sequences (Gatz, C. et al. (1992) Plant J. 2:397-404). In some embodiments of the present invention, this repressor system is similarly utilized.

A temperature-inducible gene regulatory system may also be used in the present invention, such as the exemplary TIGR system comprising a cold-inducible transactivator in the form of a fusion protein having a heat shock responsive regulator, rheA, fused to the VP16 transactivator (Weber et al,. 2003a). The promoter responsive to this fusion thermosensor comprises a *rheO* element operably linked to a minimal promoter, such as the minimal version of the human cytomegalovirus immediate early promoter. At the permissive temperature of 37°C, the cold-inducible transactivator transactivate the exemplary *rheO* -CMVₘᵢₙ promoter, permitting expression of the target gene. At 41°C, the cold-inducible transactivator no longer transactivates the *rheO* promoter.

Other embodiments useful in the present invention include the erythromycin-resistance regulon from E.coli, having repressible (E_{off}) and inducible (Eₒₙ) systems responsive to macrolide antibiotics, such as erythromycin, clarithromycin, and roxithromycin (Weber et al., 2002). The E_{off} system utilizes an erythromycin-dependent transactivator, wherein providing a macrolide antibiotic represses transgene expression. In the Eₒₙ system, the binding of the repressor to the operator results in repression of transgene expression. Therein, in the presence of macrolides gene expression is induced.

Fussenegger et al. (2000) describe repressible and inducible systems using a Pip (pristinamycin-induced protein) repressor encoded by the streptogramin resistance operon of *Streptomyces coelicolor,* wherein the systems are responsive to streptogramin-type antibiotics (such as, for example, pristinamycin, virginiamycin, and Synercid). The Pip DNA-binding domain is fused to a VP16 transactivation domain or to the KRAB silencing domain, for example. The presence or absence of, for example, pristinamycin, regulates the PipON and PipOFF systems in their respective manners, as described therein.

Another example of a transgene expression system utilizes a quorum-sensing (referring to particular prokaryotic molecule communication systems having diffusable signal molecules that prevent binding of a repressor to an operator site, resulting in derepression of a target regulon) system. For example, Weber et al. (2003b) employ a fusion protein comprisign the Streptomyces coelicolor quorum-sending receptor to a transactivating domain that regulates a chimeric promoter having a respective operator that the fusion protein binds. The expression is fine-tuned with non-toxic butyrolactones, such as SCB1 and MP 133.

In particular embodiments, multiregulated multigene therapeutic gene expression systems that are functionally compatible with one another are utilized in the present invention (see, for example, Kramer et al. (2003)). For example, in Weber et al. (2002), the macrolide-responsive erythromycin resistance regulon system is used in conjunction with a streptogramin (PIP)-regulated and tetracycline-regulated expression systems.

In a specific embodiment of the present invention, a Pol II promoter regulates expression of the transgene (such as the exemplary siRNA) (see, for example, Shinagawa and Ishii, 2003). As described in the exemplary Shinagawa and Ishhii (2003) system, a long dsRNA is generated that lacks a 5'-cap structure and a 3'-poly(A) tail, which is then processed into siRNA. The absence of the 5'-cap structure and a 3'-poly(A) tail blocks the export of the long dsRNA to the cytosol, thereby preventing induction of an interferon response. Although in this system post-transcriptional processing events are modulated by adding the *cis*-acting hammerhead ribozyme at a site downstream of the RNA start site (for removal of the 5'-cap) and a MAZ site for Pol II pausing, any means to do so would be within the scope of this invention.

### III. Transfection: Introducing the siRNA Expression System into a Cell or Organism

Transfection is the introduction of nucleic acids into recipient eukaryotic cells and the subsequent integration of the nucleic acid sequence into chromosomal DNA. Efficient transfection requires vectors, which facilitate the introduction of foreign nucleic acids into the desired cells, may provide mechanisms for chromosomal integration, and provide for the appropriate expression of the traits or proteins encoded by those nucleic acids. The design and construction of efficient, reliable, and safe vectors for cell transfection is well known to the art. In the context of the present invention, any vector which can mediate the delivery and genomic integration of the elements (a), (b), and (c) into the target cell, tissue or organism is contemplated to be within the scope of the invention.

Viruses of many types have formed the basis for vectors. Virus infection involves the introduction of the viral genome into the host cell. That property is co-opted for use as a gene delivery vehicle in viral based vectors. The viruses used are often derived from pathogenic viral species that already have many of the necessary traits and abilities to transfect cells. However, not all viruses will successfully transfect all cell types at all stages of the cell cycle. Thus, in the development of viral vectors, viral genomes are often modified to enhance their utility and effectiveness for introducing foreign gene constructs (transgenes) or other nucleic acids. At the same time, modifications may be introduced that reduce or eliminate their ability to cause disease. Thus, viral vectors derived from viruses such as retrovirus, vaccinia virus (Ridgeway, 1988; Baichwal and Sugden, 1986; Coupar *et al.,* 1988); adeno-associated virus (AAV) (Ridgeway, 1988; Baichwal and Sugden, 1986; Hermonat and Muzycska, 1984) ;and herpesviruses may be employed in the present invention. They offer several attractive features for various mammalian cells (Friedmann, 1989; Ridgeway, 1988; Baichwal and Sugden, 1986; Coupar *et al.,* 1988; Horwich *et al.,* 1990). Other viral vectors derived from viruses such as vaccinia virus (Ridgeway, 1988; Baichwal and Sugden, 1986; Coupar *et al.,* 1988), sindbis virus, cytomegalovirus and herpes simplex virus may also be employed.

Retroviral vectors are known to the art as useful in delivery of siRNA expression constructs. See, for example, the text of Devroe and Silver (2002) , which discloses that retroviruses are efficient vectors for delivery of siRNA expressing cassettes into mammalian cells. Barton and Medzhitov (2002) disclose that retroviral introduction of siRNA expression constructs results in the stable inactivation of genes in primary cells.

Lentiviruses are a subgroup of retroviruses that can infect nondividing cells owing to the karyophilic properties of their preintegration complex, which allow for its active import through the nucleopore.

### A. Lentiviral Vectors

Lentiviruses include members of the bovine lentivirus group, equine lentivirus group, feline lentivirus group, ovinecaprine lentivirus group and primate lentivirus group. The development of lentiviral vectors for gene therapy has been reviewed in Klimatcheva *et al.,* (1999). The design and use of lentiviral vectors suitable for gene therapy is described, for example, in U.S. Pat. No. 6,531,123; U.S. Pat. No. 6,207,455; and U.S. Pat. No. 6,165,782. Examples of lentiviruses include, but are not limited to, HIV-1, HIV-2, HIV-1/HIV-2 pseudotype, HIV-1/SIV, FIV, caprine arthritis encephalitis virus (CAEV), equine infectious anemia virus and bovine immunodeficiency virus. HIV-1 is preferred.

Lentiviral vectors offer great advantages for gene therapy. They integrate stably into chromosomes of target cells which is required for long-term expression. Also, they do not transfer viral genes therefore avoiding the problem of generating transduced cells that can be destroyed by cytotoxic T-cells. Additionally, they have a relatively large cloning capacity, allowing for clinical applicability. Furthermore, lentiviruses, in contrast to other retroviruses, are capable of transducing non-dividing cells. This is very important in the context of gene-therapy for tissues such as the hematopoietic system, the brain, liver, lungs and muscle. For example, vectors derived from HIV-1 allow efficient *in vivo* and *ex vivo* delivery, integration and stable expression of transgenes into cells such a neurons, hepatocytes, and myocytes (Blomer *et al.,* 1997; Kafri *et al.,* 1997; Naldini *et al.,* 1996a; 1996b).

The lentiviral genome and the proviral DNA have the three genes found in retroviruses: gag, pol and env, which are flanked by two long terminal repeat (LTR) sequences. The gag gene encodes the internal structural (matrix, capsid and nucleocapsid) proteins; the pol gene encodes the RNA-directed DNA polymerase (reverse transcriptase), a protease and an integrase; and the env gene encodes viral envelope glycoproteins. The 5' and 3' LTR's serve to promote transcription and polyadenylation of the virion RNA's. The LTR contains all other cis-acting sequences necessary for viral replication. Lentiviruses have additional genes including vif, vpr, tat, rev, vpu, nef and vpx.

Adjacent to the 5' LTR are sequences necessary for reverse transcription of the genome (the tRNA primer binding site) and for efficient encapsidation of viral RNA into particles (the Psi site). If the sequences necessary for encapsidation (or packaging of retroviral RNA into infectious virions) are missing from the viral genome, the cis defect prevents encapsidation of genomic RNA. However, the resulting mutant remains capable of directing the synthesis of all virion proteins.

Lentiviral vectors are well known in the art, see Naldini *et al.,* (1996a and 1996b); Zufferey *et al.,* (1997); Dull *et al.* (1998); U.S. Pat. Nos. 6,013,516 and 5,994,136. In general, these vectors are plasmid-based or virus-based, and are configured to carry the essential sequences for incorporating foreign nucleic acid, for selection and for transfer of the nucleic acid into a host cell.

Correspondingly, lentiviral vectors derived from human immunodeficiency virus type 1 (HIV-1) can mediate the efficient delivery, integration and long-term expression of transgenes into non-mitotic cells both *in vitro* and *in vivo* (Naldini *et al.,* 1996a; Naldini *et al.,* 1996b; Blomer *et al.,* 1997).

In the retroviral genome, a single RNA molecule that also contains all the necessary cis-acting elements carries all the coding sequences. Biosafety of a vector production system is therefore best achieved by distributing the sequences encoding its various components over as many independent units as possible, to maximize the number of crossovers that would be required to re-create an replication competent recombinants (RCR). Lentivector particles are generated by co-expressing the virion packaging elements and the vector genome in host producer cells, *e.g*. 293 human embryonic kidney cells. In the case of HIV-1-based vectors, the core and enzymatic components of the virion come from HIV-1, while the envelope protein is derived from a heterologous virus, most often VSV due to the high stability and broad tropism of its G protein. The genomic complexity of HIV, where a whole set of genes encodes virulence factors essential for pathogenesis but dispensable for transferring the virus genetic cargo, substantially aids the development of clinically acceptable vector systems.

Multiply attenuated packaging systems typically now comprise only three of the nine genes of HIV-1: gag, encoding the virion main structural proteins, pol, responsible for the retrovirus-specific enzymes, and *rev*, which encodes a post-transcriptional regulator necessary for efficient *gag* and pol expression (Dull, *et al.,* 1998). From such an extensively deleted packaging system, the parental virus cannot be reconstituted, since some 60% of its genome has been completely eliminated. In one version of an HIV-based packaging system, Gag/Pol, Rev, VSV G and the vector are produced from four separate DNA units. Also, the overlap between vector and helper sequences has been reduced to a few tens of nucleotides so that opportunities for homologous recombination are minimized.

HIV type 1 (HIV-1) based vector particles may be generated by co-expressing the virion packaging elements and the vector genome in a so-called producer cell, e.g. 293T human enbryonic kidney cells. These cells may be transiently transfected with a number of plasmids. Typically from three to four plasmids are employed, but the number may be greater depending upon the degree to which the lentiviral components are broken up into separate units. Generally, one plasmid encodes the core and enzymatic components of the virion, derived from HIV-1. This plasmid is termed the packaging plasmid. Another plasmid encodes the envelope protein(s), most commonly the G protein of vesicular stomatitis virus (VSV G) because of its high stability and broad tropism. This plasmid may be termed the envelope expression plasmid. Yet another plasmid encodes the genome to be transferred to the target cell, that is, the vector itself, and is called the transfer vector. Recombinant viruses with titers of several millions of transducing units per milliliter (TU/ml) can be generated by this technique and variants thereof. After ultracentrifugation concentrated stocks of approximately 10⁹ TU/ml can be obtained.

The vector itself is the only genetic material transferred to the target cells. It typically comprises the transgene cassette flanked by cis-acting elements necessary for its encapsidation, reverse transcription, nuclear import and integration. As has been previously done with oncoretroviral vectors, lentiviral vectors have been made that are "self-inactivating" in that they lose the transcriptional capacity of the viral long terminal repeat (LTR) once transferred to target cells (Zufferey, *et al.* 1998). This modification further reduces the risk of emergence of replication competent recombinants (RCR) and avoids problems linked to promoter interference. These vectors, or their components, are known as SIN vectors or SIN containing vectors. The SIN design is described in further detail in Zufferey et al., 1998 and U.S. Pat. No. 5,994,136 .

### B. Post-transcriptional Regulatory Element

Enhancing transgene expression may be required in certain embodiments, especially those that involve lentiviral constructs of the present invention with modestly active promoters.

One type of post-transcriptional regulatory element (PRE) is an intron positioned within the expression cassette, which can stimulate gene expression. However, introns can be spliced out during the life cycle events of a lentivirus. Hence, if introns are used as PRE's they may have to be placed in an opposite orientation to the vector genomic transcript.

Post-transcriptional regulatory elements that do not rely on splicing events offer the advantage of not being removed during the viral life cycle. Some examples are the post-transcriptional processing element of herpes simplex virus, the post-transcriptional regulatory element of the hepatitis B virus (HPRE) and the woodchuck hepatitis virus (WPRE). Of these the WPRE is most preferred as it contains an additional cis-acting element not found in the HPRE (Donello *et al.,* 1998). This regulatory element is positioned within the vector so as to be included in the RNA transcript of the transgene, but downstream of stop codon of the transgene translational unit. As demonstrated in the present invention and in Zufferey *et al.,* 1999, the WPRE element is a useful tool for stimulating and enhancing gene expression of desired transgenes in the context of the lentiviral vectors.

The WPRE is characterized and described in U.S. Pat. No. 6,136,597, . As described therein, the WPRE is an RNA export element that mediates efficient transport of RNA from the nucleus to the cytoplasm. It enhances the expression of transgenes by insertion of a cis-acting nucleic acid sequence, such that the element and the transgene are contained within a single transcript. Presence of the WPRE in the sense orientation was shown to increase transgene expression by up to 7 to 10 fold. Retroviral vectors deliver sequences in the form of cDNAs instead of complete intron-containing genes as introns are generally spliced out during the sequence of events leading to the formation of the retroviral particle. Introns mediate the interaction of primary transcripts with the splicing machinery. Because the processing of RNAs by the splicing machinery facilitates their cytoplasmic export, due to a coupling between the splicing and transport machineries, cDNAs are often inefficiently expressed. Thus, the inclusion of the WPRE in a vector results in enhanced expression of transgenes.

The introduction of foreign nucleic acids into the nucleus of a cell requires importation of the nucleic acids into the nucleus through the nuclear membrane. Lentiviruses utilize an active nuclear import system, which forms the basis of their ability to replicate efficiently in non-dividing cells. This active import system relies upon a complex series of events including a specific modality for reverse transcription. In particular, in HIV-1, the central polypurine tract (cPPT), located within the *pol* gene, initiates synthesis of a downstream plus strand while plus strand synthesis is also initiated at the 3' polypurine tract (PPT). After strand transfer of the short DNA molecule, the upstream plus strand synthesis will initiate and proceed until the center of the genome is reached. At the central termination sequence (cTS) the HIV-1 reverse transcriptase is ejected, (released from its template), when functioning in a strand displacement mode. (Charneau, *et al*., 1994) The net result is a double stranded DNA molecule with a stable flap, 99 nucleotides in length at the center of the genome. This central "flap" facilitates nuclear import. (Zennou, *et al.,* 2000).

### IV. Transgenic Mice and Other non-human Transgenic Animals

The methods used for generating transgenic mice are well known to one of skill in the art. For example, one may use the manual entitled "Manipulating the Mouse Embryo", 1986. See for example, Leder and Stewart, U.S. Pat. No. 4,736,866 for methods for the production of a transgenic mouse. Other examples include the following U.S. Patents : U.S. Pat. No. 6,025,539, relating to IL-5 transgenic mouse; U.S. Pat. No. 6,023,010, Transgenic non-human animals depleted in a mature lymphocytic cell-type; U.S. Pat. No. 6,018,098, In vivo and *in vitro* model of cutaneous photoaging; U.S. Pat. No. 6,018,097, Transgenic mice expressing human insulin; U.S. Pat. No. 6,008,434, Growth differentiation factor-11 transgenic mice; U.S. Pat. No. 6,002,066; H2-M modified transgenic mice; U.S. Pat. No. 5,994,618, Growth differentiation factor-8 transgenic mice; U.S. Pat. No. 5,986,171, Method for examining neurovirulence of polio virus, U.S. Pat. No. 5,981,830, Knockout mice and their progeny with a disrupted hepsin gene; U.S. Pat. No. 5,981,829, DELTA.Nur77 transgenic mouse; U.S. Pat. No. 5,936,138; Gene encoding mutant L3T4 protein which facilitates HIV infection and transgenic mouse expressing such protein; U.S. Pat. No. 5,912,411, Mice transgenic for a tetracycline-inducible transcriptional activator; U.S. Pat. No. 5,894,078, Transgenic mouse expressing C-100 app.

It is well known in the art that it is possible to carry out the genetic transformation of a zygote (and the embryo and mature organism which result therefrom) by placing or inserting exogenous genetic material into the nucleus of the zygote or to any nucleic genetic material which ultimately forms a part of the nucleus of the zygote. The genotype of the zygote and the organism which results from a zygote will include the genotype of the exogenous genetic material. Additionally, the inclusion of exogenous genetic material in the zygote results in a phenotype expression of the exogenous genetic material.

The genotype of the exogenous genetic material is expressed upon the cellular division of the zygote. However, the phenotype expression, e.g., the production of a protein product or products of the exogenous genetic material, or alterations of the zygote's or organism's natural phenotype, will occur at that point of the zygote's or organism's development during which the particular exogenous genetic material is active. Alterations of the expression of the phenotype include an enhancement or diminution in the expression of a phenotype or an alteration in the promotion and/or control of a phenotype, including the addition of a new promoter and/or controller or supplementation of an existing promoter and/or controller of the phenotype.

The genetic transformation of various types of organisms, is disclosed and described in detail in U.S. Pat. No. 4,873,191. The genetic transformation of organisms can be used as an in vivo analysis of gene expression during differentiation and in the elimination or diminution of genetic diseases by either gene therapy or by using a transgenic non-human mammal as a model system of a human disease. This model system can be used to test putative drugs for their potential therapeutic value in humans.

The exogenous genetic material can be placed in the nucleus of a mature egg. It is preferred that the egg be in a fertilized or activated (by parthenogenesis) state. After the addition of the exogenous genetic material, a complementary haploid set of chromosomes (*e.g*., a sperm cell or polar body) is added to enable the formation of a zygote. The zygote is allowed to develop into an organism such as by implanting it in a pseudopregnant female. The resulting organism is analyzed for the integration of the exogenous genetic material. If positive integration is determined, the organism can be used for the in vivo analysis of the gene expression, which expression is believed to be related to a particular genetic disease.

Attempts have been made to study a number of different types of genetic diseases utilizing such transgenic animals. See, for example, WO89/06689 and WO89/06693 relating to the study of Alzheimer's disease.

Embryonal target cells at various developmental stages can be used to introduce transgenes. Different methods are used depending on the stage of development of the embryonal target cell. The zygote is the best target for micro-injection. In the mouse, the male pronucleus reaches the size of approximately 20 micrometers in diameter which allows reproducible injection of 1-2 pl of DNA solution. The use of zygotes as a target for gene transfer has a major advantage in that in most cases the injected DNA will be incorporated into the host gene before the first cleavage (Brinster, *et al.,* 1985). As a consequence, all cells of the transgenic non-human animal will carry the incorporated transgene. This will in general also be reflected in the efficient transmission of the transgene to offspring of the founder since 50% of the germ cells will harbor the transgene. Microinjection of zygotes is the preferred method for incorporating transgenes.

Retroviral infection can also be used to introduce transgene into a non-human animal. The developing non-human embryo can be cultured *in vitro* to the blastocyst stage. During this time, the blastomeres can be targets for retroviral infection (Jaenich, 1976). Efficient infection of the blastomeres is obtained by enzymatic treatment to remove the zona pellucida (Hogan, *et al.,* 1986). The viral vector system used to introduce the transgene is typically a replication-defective retrovirus carrying the transgene, Jahner, *et al.* (1985); Van der Putten, *et al.* (1985). Transfection is easily and efficiently obtained by culturing the blastomeres on a monolayer of virus-producing cells (Van der Putten, supra; Stewart, *et al.,* 1987). Alternatively, infection can be performed at a later stage. Virus or virus-producing cells can be injected into the blastocoele (Jahner, 1982). Most of the founders will be mosaic for the transgene since incorporation occurs only in a subset of the cells which formed the transgenic non-human animal. Further, the founder may contain various retroviral insertions of the transgene at different positions in the genome which generally will segregate in the offspring. In addition, it is also possible to introduce transgenes into the germ line, albeit with low efficiency, by intrauterine retroviral infection of the midgestation embryo (Jahner, 1982).

A third type of target cell for transgene introduction is the non-human embryonal stem cell (ES). ES cells are obtained from pre-implantation embryos cultured *in vitro* and fused with embryos (Evans *et al.,* 1981; Bradley *et al.,* 1984; Gossler *et al.,* 1986; Robertson *et al.,* 1986). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, (1988.)

As used herein, a "transgene" is a DNA sequence introduced into the germline of a non-human animal by way of human intervention such as by way of the above described methods.

Thus, in one particular aspect of the invention there are non-human transgenic animals having an exemplary transgene comprising a polynucleotide sequence encoding a tetracycline-controllable or tetracycline analog-controllable DNA-binding domain operably fused to the KRAB repression domain of the invention or having an exemplary transgene encoding a siRNA operably linked to a promoter and a sequence bindable by the afore-mentioned fusion gene product. Double transgenic animals having both transgenes (*i.e*., the transgene encoding the tetracycline-controllable or tetracycline analog-controllable fusion protein and the transgene comprising a siRNA linked to a promoter responsive to the fusion protein) are also encompassed by the invention. In one embodiment, the transgenic animal is a mouse. In other embodiments, the transgenic animal is a cow, a goat, a sheep or a pig. Transgenic animals of the invention can be made, for example, by introducing a DNA molecule encoding the afore-mentioned exemplary transgenes into a fertilized oocyte, implanting the fertilized oocyte in a pseudopregnant foster mother, and allowing the fertilized oocyte to develop into the non-human transgenic animal to thereby produce the non-human transgenic animal. Double transgenic animals can be created by appropriate mating of single transgenic animals. Expression of a siRNA operably linked to a promoter responsive to a tetracycline or tetracycline analog-inducible fusion protein that regulates the promoter in a double transgenic animal of the invention can be inhibited by administering tetracycline or a tetracycline analog to the animal.

Another particular aspect of the invention relates to non-human transgenic animals having a transgene encoding a tetracycline or a tetracycline analog fusion protein of the invention, wherein the transgene is integrated by homologous recombination at a predetermined location within a chromosome within cells of the animal (also referred to herein as a homologous recombinant animal). The homologous recombinant animal can also have a second transgene encoding a siRNA operably linked to a promoter responsive to the tetracycline or a tetracycline analog-controllable fusion protein. The second transgene can be introduced randomly or, alternatively, at a predetermined location within a chromosome (*e.g*., by homologous recombination).

A non-human transgenic animal of the invention having tTR-KRAB-coding sequences integrated at a predetermined location within chromosomal DNA of cells of the animal can be created by introducing a targeting vector of the invention into a population of embryonic stem cells under conditions suitable for homologous recombination between the DNA encoding the tTR-KRAB and chromosomal DNA within the cell, selecting an embryonic stem cell in which DNA encoding the tTR-KRAB has integrated at a predetermined location within the chromosomal DNA of the cell, implanting the embryonic stem cell into a blastocyst, implanting the blastocyst into a pseudopregnant foster mother and allowing the blastocyst to develop into the non-human transgenic animal to thereby produce the non-human transgenic animal.

### A. Conditional non-human Transgenic Animals

The present invention further contemplates conditional transgenic or knockdown non-human animals, such as those produced using recombination methods. Bacteriophage P1 Cre recombinase and flp recombinase from yeast plasmids are two non-limiting examples of site-specific DNA recombinase enzymes which cleave DNA at specific target sites (lox P sites for cre recombinase and frt sites for flp recombinase) and catalyze a ligation of this DNA to a second cleaved site. A large number of suitable alternative site-specific recombinases have been described, and their genes can be used in accordance with the method of the present invention. Such recombinases include the Int recombinase of bacteriophage λ (with or without Xis) (Weisberg *et. al.,* 1983), ); TpnI and the β-lactamase transposons (Mercier *et al.,* 1990); the Tn3 resolvase (Flanagan and Fennewald, 1989; Stark *et al.,* 1989); the yeast recombinases (Matsuzaki *et al.,* 1990); the B. subtilis SpoIVC recombinase (Sato *et al.,* 1990); the Flp recombinase (Schwartz and Sadowski, 1989; Parsons *et al.,* 1990; Golic and Lindquist, 1989; Amin *et al.,* 1990); the Hin recombinase (Glasgow *et al.,* 1989); immunoglobulin recombinases (Malynn *et al.,* 1988); and the Cin recombinase (Haffter and Bickle, 1988; Hubner *et al.,* 1989). Such systems are discussed (Echols, 1990; de Villartay, 1988; Craig, 1988; Poyart-Salmeron *et al.,* 1989; Hunger-Bertling *et al.,* 1990; and Cregg and Madden, 1989).

Of particular interest in the present invention is the Cre recombinase. Cre has been purified to homogeneity, and its reaction with the loxP site has been extensively characterized (Abremski and Hess, 1984)). Cre protein has a molecular weight of 35,000 and can be obtained commercially from New England Nuclear/DuPont. The cre gene (which encodes the Cre protein) has been cloned and expressed (Abremski *et al*., 1983)). The Cre protein mediates recombination between two loxP sequences (Stemberg *et al*., 1981), which may be present on the same or different DNA molecule. Because the internal spacer sequence of the loxP site is asymmetrical, two loxP sites can exhibit directionality relative to one another (Hoess and Abremski, 1984). Thus, when two sites on the same DNA molecule are in a directly repeated orientation, Cre will excise the DNA between the sites (Abremski *et al.,* 1983). However, if the sites are inverted with respect to each other, the DNA between them is not excised after recombination but is simply inverted. Thus, a circular DNA molecule having two loxP sites in direct orientation will recombine to produce two smaller circles, whereas circular molecules having two loxP sites in an inverted orientation simply invert the DNA sequences flanked by the loxP sites. In addition, recombinase action can result in reciprocal exchange of regions distal to the target site when targets are present on separate DNA molecules.

Recombinases have important application for characterizing gene function in knockout models. When the constructs described herein are used to disrupt limulus clotting factor protease-like genes, a fusion transcript can be produced when insertion of the positive selection marker occurs downstream (3') of the translation initiation site of the limulus clotting factor protease-like gene. The fusion transcript could result in some level of protein expression with unknown consequence. It has been suggested that insertion of a positive selection marker gene can affect the expression of nearby genes. These effects may make it difficult to determine gene function after a knockout event since one could not discern whether a given phenotype is associated with the inactivation of a gene, or the transcription of nearby genes. Both potential problems are solved by exploiting recombinase activity. When the positive selection marker is flanked by recombinase sites in the same orientation, the addition of the corresponding recombinase will result in the removal of the positive selection marker. In this way, effects caused by the positive selection marker or expression of fusion transcripts are avoided.

### B. Transgenic Knockdown Mice

In some aspects, the present invention contemplated a method of creating a transgenic animal capable of exhibiting conditional knockdown of a target gene, which may be accomplished in a tissue-specific manner, though it need not be. The 'knocking down' of a gene is implemented by interfering with the transcription of the mutant gene to a harmful protein. This methodology is applied in the creation of transgenic mice in which inherited RNAi lowers or silences the expression of a target gene, producing a stable "gene knockdown."

To adapt RNAi for the study of gene function in mice, genetic engineering was used to create mouse embryonic stem cells in which RNAi was targeted to a particular gene (Carmell *et al.,* 2003). This was based on a previous study in which silencing a gene of interest through RNAi was efficiently achieved by engineering a second gene that encoded short hairpin RNA molecules corresponding to the gene of interest (Carmell *et al.,* 2003). The stem cells were injected into mouse embryos, and chimeric animals were born. Matings of these chimeric mice produced offspring that contained the genetically engineered RNAi-inducing gene in every cell of their bodies.

It was observed from examination of the tissues from the transgenic mice, that the expression of the gene of interest was significantly reduced throughout the organism *(e.g*. liver, heart, spleen). Such a reduction in gene expression is called a "gene knockdown" to distinguish it from traditional methods that involve "gene knockouts" or the complete deletion of a DNA segment from a chromosome.

One advantage of this RNAi-based gene knockdown strategy, is that the strategy can be modified to silence the expression of genes in specific tissues, and it can be designed to be switched on and off at any time during the development or adulthood of the animal.

### V. Therapeutic Applications

The invention is widely applicable to a variety of situations where it is desirable to be able to regulate the level of gene expression, such as by turning gene expression "on" and "off", in a rapid, efficient and controlled manner without causing pleiotropic effects or cytotoxicity. The invention may be particularly useful for gene therapy purposes in humans, in treatments for either genetic or acquired diseases. The general approach of gene therapy involves the introduction of one or more nucleic acid molecules into cells such that one or more gene products encoded by the introduced genetic material are produced in the cells to restore or enhance a functional activity. For reviews on gene therapy approaches Anderson, et al. (1992; Miller et al. (1992); Friedmann et al. (1989); and Cournoyer *et al.* (1990). However, current gene therapy vectors typically utilize constitutive regulatory elements which are responsive to endogenous transcriptions factors. These vector systems do not allow for the ability to modulate the level of gene expression in a subject. In contrast, the regulatory system of the invention provides this ability.

To use the system of the invention for gene therapy purposes, at least one DNA molecule is introduced into cells of a subject in need of gene therapy (*e.g*., a human subject suffering from a genetic or acquired disease) to modify the cells. The cells are modified to comprise: 1) nucleic acid encoding an inducible regulator of the invention in a form suitable for expression of the inducible regulator in the host cells; and 2) an siRNA (*e.g*., for therapeutic purposes) operatively linked to an inducible regulator-responsive promoter (*e.g*., at least one tet operator sequence(s) and, optionally, with a minimal promoter). A single DNA molecule encoding components of the regulatory system of the invention can be used, or alternatively, separate DNA molecules encoding each component can be used. The cells of the subject can be modified *ex vivo* and then introduced into the subject or the cells can be directly modified *in vivo* by conventional techniques for introducing nucleic acid into cells. Expression of the siRNA of interest in the cells of the subject is stimulated in the presence of Tc or a Tc analog, whereas expression is inhibited in the absence of Tc or a Tc analog to the patient. The level of gene expression can be varied depending upon which particular Tc analog is used as the inducing agent, in some embodiments. Additionally, expression of the siRNA can be adjusted according to the medical needs of the individual, which may vary throughout the lifetime of the individual. Thus, the regulatory system of the invention offers the advantage over constitutive regulatory systems of allowing for modulation of the level of gene expression depending upon the requirements of the therapeutic situation.

Genes of particular interest to be knocked down or knocked out in cells of a subject for treatment of genetic or acquired diseases include those encoding a deleterious gene product, such as an abnormal protein. Examples of non-limiting specific diseases include hyperthyroidism, a disease condition associated with a hypersecretion defect, and Alzheimer's Syndrome.

Gene therapy applications of particular interest in cancer treatment include, for example, oncogenes, such as ABLI, BLC1, BCL6, CBFA1, CBL, CSFIR, ERBA, ERBB, EBRB2, ETS1, ETS1, ETV6, FGR, FOX, FYN, HCR, HRAS, JUN, KRAS, LCK, LYN, MDM2, MLL, MYB, MYC, MYCL1, MYCN, NRAS, PIM1, PML, RET, SRC, TAL1, TCL3 and YES. A skilled artisan recognizes that treatment of cancer using methods and compositions of the present invention may be used in combination with other forms of cancer treatment, such as surgery, chemotherapy, radiation, gene therapy, immunotherapy, and so forth. Types of cancer for treatment include the non-limiting examples of gliosarcoma, breast cancer, lung cancer, brain cancer, melanoma, prostate cancer, ovarian cancer, pancreatic cancer, liver cancer, colon cancer, cervical cancer, bladder cancer, spleen cancer, head and neck cancer, and/or bone cancer.

The present invention can be applied to develop autologous or allogeneic cell lines for therapeutical purposes. For example, gene therapy applications of particular interest in cell and/or organ transplantation are utilized with the present invention. In exemplary embodiments, downregulation of transplantation antigens (such as, for example, by downregulation of beta2-microglobulin expression *via* siRNA) allows for transplantation of allogeneic cells while minimizing the risk of rejection by the patient's immune system. The present invention would allow for a switch off of the RNAi in case of adverse effects (*e.g*. uncontrollable replication of the transplanted cells).

Cells types that can be subjected to the present invention include hematopoietic stem cells, myoblasts, hepatocytes, lymphocytes, airway epithelium, skin epithelium, islets, dopaminergic neurons, keratinocytes, and so forth. For further descriptions of cell types, genes and methods for gene therapy see *e.g*., Wilson *et al.* (1988); Armentano *et al.* (1990); Wolff *et al.* (1990); Chowdhury *et al.* (1991); Ferry *et al.* (1991); Wilson *et al.* (1992); Quantin *et al.* (1992); Dai *et al.* (1992); van Beusechem *et al.* (1992); Rosenfeld *et al.* (1992); Kay *et al.* (1992); Cristiano *et al.* (1993); Hwu *et al*. (1993); and Herz and Gerard (1993).

Disclosed is a method of treating any disease condition amenable to treatment with a siRNA. In specific embodiments, the method comprises preparing a polynucleotide construct having a region encoding an siRNA that is operably linked to an externally controllable promoter, wherein the siRNA encoded by the construct is for the treatment of the disease condition, and furthermore externally regulating the expression of the siRNA through the externally controllable promoter.

The disease may, for example, be a hyperproliferative disorder, such as cancer, and specific exemplary cancers include gliosarcoma, breast cancer, lung cancer, brain cancer, melanoma, prostate cancer, ovarian cancer, pancreatic cancer, liver cancer, colon cancer, cervical cancer, bladder cancer, spleen cancer, head and neck cancer, or bone cancer.

In other embodiments, the disease condition relates to hypersecretion defects, such as those associated with hypersecretion of at least one hormone. Specific examples include hypersecretion of thyroxine (such as with Graves' disease), hypersecretion of glucocorticoids (such as with Cushing's Syndrome), hypersecretion of growth hormone (such as with gigantism or acromegaly), hypersecretion of insulin, hypersecretion of mineral corticoids (such as with aldosteronism), hypersecretion of androgens (such as with Androgenital. Syndrome in females), hypersecretion of cstrogens (such as an increased incidence of breast and/or ovarian cancer in females and gynecomastia in male), or hypersecretion of epinephrine and/or norepinephrine. Whereas current therapies for hypersecretion defects may comprise drastic therapeutic measures, such as removal of an adrenal gland, for example, an advantage to the present invention is the ability to fine tune the hypersecretion therapy through specialized regulation in accordance with the methods and compositions described herein.

### Control of Immunological Recognition of a Cell

To treat organ: disease and organ failure, the use of allogeneic, or non-self, transplantation tissue has become increasingly important in medicine. The use of allografts, however, is limited by the frequent rejection of the graft tissue by the recipient host, because of antigenic differences between the donor and recipient.

The antigenic differences between individual members of the same species are referred to as "alloantigens." When alloantigens are involved in rejection of allogeneic tissue grafts, they are referred to as "histocompatibility antigens." The terms "major histocompatibility antigens" and "major histocompatibility complex" (MHC) refer to the products of a single closely linked region of genes. These MHC gene products are displayed on cell surfaces and are an important barrier to successful allotransplantation.

A skilled artisan recognizes that a key to the immune defensive mechanism is the T-cell. T-cells have been found to be restricted in that they respond to an antigen in relation to one or a few specific transplantation antigens associated with their natural host. *In vitro,* T-cells from one haplotype host respond to an antigen in relation to a transplantation antigen of a different haplotype host. The T-cell receptor repertoire appears to be narrower than the B-cell immunoglobulin repertoire. In addition, rather than directly binding to the antigen, the T-cell receptor appears to require concomitant binding to an antigenic epitope and a transplantation antigen. It is known in the art that an immunogenic transplantation antigen comprises a component of the major histocompatibility complex.

The transplantation antigens are divided into two classes, Class I and Class II, where the former class of antigens is relatively ubiquitous on host cells, while the latter class is relatively limited to lymphocytes, macrophages, and dendritic cells. Different T-cells appear to be activated in relation to one or the other class of transplantation antigen. In the main, the nature of the activity of a T-cell will vary with the class of the transplantation antigen to which it is complementary.

In effect it appears that a T-cell clone recognizes a specific antigen in conjunction with a specific transplantation antigen allele. Furthermore, variation in the antigen sequence, affects the nature of the response when the T-cell, antigen, and antigen presenting cell are brought together in culture. Depending upon the nature of the change, all three possibilities are encountered, namely, no change, increased stimulation or decreased stimulation.

In view of the above described events, it would be of substantial interest to be able to modify the immune response *in vivo* and *in vitro,* where one could provide stimulation or inactivation of a particular immune response. In this manner, the natural response to a particular event could be modulated, either by activating particular lymphocytes to enhance the protective response or by deactivating particular lymphocytes to diminish or prevent an immune response.

The downregulation of a transplantation antigen may mask the immunogenicity of the transplant. Disclosed are methods and compositions for controlling the ability of a cell to be recognized immunologically. As such, methods are disclosed that generate at least one cell that can be transplanted without the risk of eliciting an immune response. In a specific aspect to the invention, one or more cells are generated wherein MHC I knockdown occurs in a controllable manner. If MHC I knockdown occurs in a cell, at least the majority and in some embodiments substantially all of the MHC antigens are absent on the cell. As such, the histocompatibility antigen being absent on the cell renders the cell unrecognizable as foreign.

A skilled artisan recognizes that the controllable nature of the methods described, such as by removing the externally applied agent, abstaining from adding further externally applied agent, adding externally applied agent, adding more externally applied agent, and so forth, is useful in the case of a desired need to cease using the invention. Thus, the ability to mask cells from an immune response that otherwise would not be easily transplantable is an advantage to the invention, and the manner of utilizing the invention in a reversible fashion is also particularly beneficial.

The entire or substantially entire MHC I complex may be absent upon regulation of a gene product that controls the ability to be recognized immunologically. Examples include beta2-microglobulin, molecules that complex with beta2-microglobulin, and/or molecules with a similar function as beta2-microglobulin.

Transplantation antigens from either Class I or Class II, or both, may be downregulated with methods and compositions described. MHC I transplantation antigens may be downregulated, such as the exemplary beta2-microglobulin. Other examples of transplantation antigens include any of the HLAs, including HLA-C, HLA-G, and HLA-DQ; H-Y; P35B; Kdm4 and Kdm5, TL, P198, P91A; H-2Kb, and so forth.

Cells useful in this context include, for example, stem cells, such as non-human embryonic stem cells, islet cells; hepatocytes, dopaminergic neurons, keratinocytes, or a mixture thereof. A stent cell, such as an embryonic stem cell, may be modified to employ the present invention, such as by knocking down a transplantation antigen, for example beta2-microglobulin. The modified stem cell then differentiates. Modified stem cells either before or after differentiation, or both, may be transplanted.

### VI. Animal Models of Human Disease

The methods and compositions described can be used alone or in combination to stimulate or inhibit expression of specific genes in animals to mimic the pathophysiology of human disease, thereby creating animal models of human disease. For example, in a host animal, a gene of interest thought to be involved in a disease can be the target of a siRNA as described herein. Such an animal can be, for example, mated to a second animal carrying one or more transgenes for an inducible fusion protein that regulates expression of the siRNA to create progeny that carry both a tetracycline or tetracycline analog-regulated fusion protein(s) gene and a siRNA which expression is affected thereby. Expression of the gene targeted by the siRNA can be downmodulated using the tetracycline or tetracycline analog-regulated fusion protein to examine the relationship between gene expression and the disease. Such an approach may be advantageous over gene "knock out" by homologous recombination to create animal models of disease, since the tet-regulated system, as an exemplary embodiment, described herein allows for control over both the levels of expression of the gene of interest and the timing of when gene expression is down- or up-regulated.

### VIII. Examples

The following examples are included to demonstrate preferred embodiments of the invention. It should be appreciated by those of skill in the art that the techniques disclosed in the examples that follow represent techniques discovered by the inventor to function well in the practice of the invention, and thus can be considered to constitute preferred modes for its practice.

### EXAMPLE 1

### Doxyclyline-inducible Regulation of GFP Expression by tKRAB-mediated Repression of siRNA Production

In the present example of the system, elements (a), (b) and (c) are incorporated into a lentiviral vector. The transrepressor (tTR-KRAB) is composed of the DNA binding domain of the tetracycline repressor tTR fused to the KRAB repression domain of human Kox-1. tTR-KRAB expression is controlled by a constitutive EF-1α promoter, *tetO* (tetracycline operator) sequence, U6 or H1 promoter, sihRNA are inserted into the U3 region of the 3' long terminal repeat of the lentiviral vector. In the target cells, this element will be present in both LTR of the integrated provirus owing to the modalities of reverse transcription, which duplicates the U3 region of the 3'LTR (FIG. 1).

In the absence of doxycycline tTR-KRAB binds to *tetO* and blocks sihRNA synthesis thus permitting expression of the sihRNA target gene (for instance a cellular gene of interest). In the presence of doxycycline tTR-KRAB is released from *tetO,* sihRNAs are produced and expression of the target gene is inhibited.

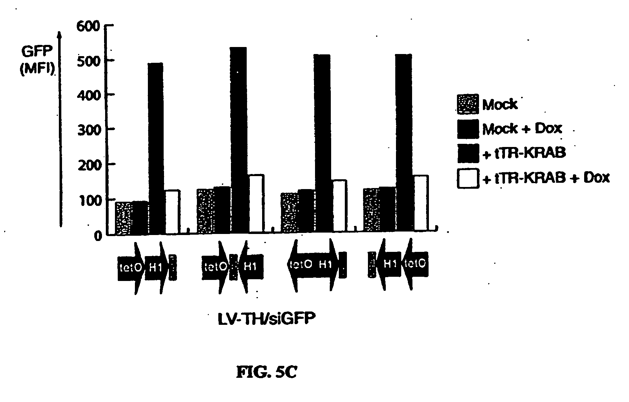

Data presented in FIG. 2 (and similarly shown in FIG. 5C) illustrate the regulated expression of a GFP marker protein using the above-described system. A HeLa cell line that constitutively expresses GFP (Hela-GFP) was cotransduced with a lentivector carrying *tetO*-U6 and GFP-specific siRNA (pNGFR-siGFP/*tetO*, or pNGFR-siGFP/*tetO*inv, or pNGFR-siGFPinv/*tetO*, or pNGFR-siGFPinv/*tetO*inv), and a lentivector carrying the tTR-KRAB cDNA under the transcriptional control of EF-1α promoter (pWPXL-KRAB). In the presence of doxycycline GFP expression was significantly inhibited, reflecting the release of the tTR-KRAB from the *tetO* and GFP-specific shRNA synthesis. In contrast, in the absence of doxycycline, tTR-KRAB binding to *tetO* led to repression of sihRNA synthesis thus allowing GFP expression. This data demonstrates that the system of the invention can be used for the efficient and specific regulation of an endogenous or exogenous gene. The GFP reporter used here is equivalent to an endogenous gene since it is integrated in the cell chromosomes. Furthermore, endogenous gene control via siRNA expression from viral vectors is known to be effective (*e.g*. Devroe and Silver, 2002).

### EXAMPLE 2

### Material and Methods

The following materials and methods were used for Example 3 and can be used to implement embodiments of the invention described herein.

**Vector construction.** Vectors were constructed using standard cloning procedures. pSUPER and pSUPER-p53 constructs were described previously (Brummelkamp *et al*., 2002). pLV-H was constructed by inserting the H1 promoter from pSUPER into the 3' LTR of pWPXL. To construct pLVTH the *tetO* cassette was excised from pUHD13-3 and cloned into pLV-H, upstream of the H1 promoter.

Finally, the H1 promoter cassette in pLV-H and pLV-TH was replaced by H1-siRNA cassette excised from pSUPER-siRNA, generating pLV-H/siRNA and pLV-TH/siRNA respectively. The sequence encoding tTR-KRAB was cloned into pWPXL replacing GFP marker (pLV-tTRKRAB), or as part of a bicistronic unit also encoding dsRed, using the encephalomyocarditis virus 5' internal ribosome entry site (IRES).

**Cell culture and transduction with lentiviral vectors.** 293T, Hela and MCF-7 cell lines were cultured in DMEM supplemented with 10% fetal calf serum. All recombinant lentiviruses were produced using transient transfection of 293T cells according to standard protocols (Zufferey *et al*., 1997). Briefly, subconfluent 293T were cotransfected by 20 µg of a plasmid vector, 15 µg of pCMV-ΔR8.91 and 5 µg of pMD2G-VSVG using calcium phosphate-precipitation. After 16 hrs, the medium was changed and recombinant lentivectors were harvested 24 hrs later.

To analyze the regulation of GFP a Hela cell clone carrying a single copy of the WPXLGFP provirus (Hela-GFP) was used. For transduction, Hela-GFP, MCF-7 or Hela cells were plated on 24-well plate (20×10⁴ cells/well) and after 16 hrs medium containing recombinant lentivectors was added. Following 16 hrs of incubation the cells were washed, split and doxycycline was added to half of the transduced cells at a final concentration of 5 µg/ml. Five days later the cells were harvested and analyzed by FACS.

**Western Blot Analysis.** Cell extracts were prepared in RIPA lysis buffer (25 mM Tris pH 7.5, 1% Triton X-100, 0.5% sodium deoxycholate, 5 mM EDTA, 150 mM NaCl) containing a cocktail of protease inhibitors (Sigma). The protein samples (10 µg) were separated on 4-20% gradient PAGE-SDS gel, electroblotted to polyvinylidene fluoride membranes (Perkin Elmer) and exposed to antibodies against p53 (Santa Cruz Biotechnology), Lamin A/C (Santa Cruz Biotechnology), GFP (Clontech) and actin (Calbiochem). Antibodies conjugated with horseradish peroxidase (Amersham) and enhanced chemiluminescence (ECL; Amersham) was used for detection.

**FACS analysis.** Harvested Hela-GFP cells transduced with lentivectors carrying ΔNGFR cDNA were incubated with monoclonal antibody specific for human NGFR (Becton Dickinson Pharmingen) labeled with phycoerythrin (NGFR-PE), washed twice and analyzed using FACSscan (Becton Dickinson) for green (GFP) and red (NGFR-PE) fluorescence. MCF-7 and Hela cells cotransduced with LV-THsi/p53 or LV-THsi/lamin and pLV-tTR-KRAB-Red and cultured in presence or absence of dox were harvested and analyzed using FACSscan for green and red (dsRed) fluorescence.

**Immunofluorescence.** MCF-7 and Hela cells cotransduced with LV-THsi/p53 or LVTHsi/ lamin and pLV-tTR-KRAB-Red and cultured five days in the presence or absence of dox were fixed with methanol (10 min/-20 °C), blocked with PBS/1% BSA and stained with antibodies against p53 (Santa Cruz Biotechnology) or Lamin A/C (Santa Cruz Biotechnology), using secondary antibodies conjugated with Alexa 633 (Molecular Probes) for detection. Images were acquired using three-color confocal microscopy (LSM 510, Carl Zeiss) and analyzed using Zeiss software.

### EXAMPLE 3

### Results and Discussion

This study takes advantage of a tetracycline-controlled hybrid protein, tTR-KRAB, in which the tetracycline repressor (tTR) from E. coli *Tn10* is fused to the KRAB domain of human Kox1 (Deuschle *et al.,* 1995; Gossen and Bujard, 1992). KRAB is an approximately 75 amino-acid-long transcriptional repression module found in many zinc finger-containing proteins, which can suppress, in an orientation-independent manner, both pol II- and and pol III-mediated transcription within a distance of up to 3kb from its binding site, presumably by triggering the formation of heterochromatin (Bellefroid *et al*., 1991; Deuschle *et al.,* 1995; Margolin *et al.,* 1994; Moosmann et al, 1997; Senatore *et al.,* 1999). When linked to the DNA-binding domain of tTR, KRAB can modulate transcription from an integrated promoter juxtaposed with *tet* operator (*testO*) sequences (6). In the absence of doxycycline (dox), tTR-KRAB binds specifically to *tetO* and suppresses the activity of the nearby promoter. Conversely, in the presence of doxycycline, tTR-KRAB is sequestered away from *tetO,* thus permitting gene expression (Deuschle *et al.,* 1995).

HIV-1-derived lentiviral vectors (LV) were used as delivery vehicles as this provides for a system applicable to a broad variety of cellular targets, be it *ex vivo* (cell lines, primary cells including stem cells, fertilized oocytes, blastocysts) or in *vivo* (*e.g.* brain, liver) (Jacque *et al.,* 2002; Miyoshi *et al.,* 1999; Naldini *et al.,* 1996a; 1996b; Pfeifer *et al.,* 2002; Qin *et al.,* 2003; Rubinson *et al.,* 2003; Tiscornia *et al.,* 2003); and because *tetO*-linked transcriptional units are repressed by tTR-KRAB only when integrated in the genome. The tTR-KRAB cDNA was expressed from the ubiquitously active EF1-a promoter as part a bicistronic transcript also producing the dsRed marker (FIG. 3A, LV-tTR-KRAB). The regulated siRNA vectors were constructed by inserting a *tetO*-H1 promoter-siRNA cassette into the U3 region of the 3' long terminal repeat (LTR) of a self-inactivating lentiviral vector (FIG. 3A, LV-THsi). During reverse transcription, the vector RNA 3' U3 region serves as the template for the synthesis of its 5' DNA homologue, so that the *tetO*-H1-siRNA cassette is duplicated in the integrated provirus (FIG. 3B). This double-copy configuration was chose to obtain higher rates of siRNA synthesis. Sequences encoding siRNA hairpin precursors were designed as described (Brummelkamp *et al.,* 2002). Control vectors carried either a constitutively active H1-siRNA cassette (LV-Hsi), or the H1- or *tetO*-H1 transcriptional elements without downstream siRNA coding sequence (LV-H and LV-TH, respectively). All siRNA and control vectors also encoded a marker gene downstream of an internal EF1-a promoter. It was predicted (FIG. 4A) that cells co-transduced with LV-THsi and LVtTR-KRAB would normally express the gene targeted by the siRNA when maintained in the absence of dox, owing to tTR-KRAB-mediated suppression of siRNA synthesis. In contrast, addition of the drug would relieve this inhibition and allow for target gene downregulation (FIG. 4B). Expression of the internal marker gene would also be subjected to conditional tTR-KRAB repression, thus providing an internal monitoring device. FIG. 5C illustrates that repression of polymerase III-mediated transcription of tTR-KRAB in a lentiviral vector is independent of orientation of the *tetO* element or polymerase III promoter (HI) to each other as well as to the lentiviral vector.

In a first series of experiments, the ability of this system to regulate the production of GFP in HeLa cells stably expressing this fluorophore was investigated (FIG. 5A). Vectors were used at a multiplicity of infection of 10 to ensure good rates of (co-) transduction. HeLa-GFP cells transduced with the empty LV-TH vector remained strongly GFP positive irrespective of their culture conditions. In contrast, cells transduced with the constitutively active LV-Hsi/GFP vector exhibited a strong downregulation of the marker. In cells transduced with the controllable LV-THsi/GFP vector, GFP expression was observed only in the presence of tTR-KRAB and in the absence of dox (FIG. 5A). Correspondingly, in the absence of drug, tTR-KRAB suppressed the expression of the vector's ΔNGFR internal reporter gene (FIG. 5B). As expected, the tTR-KRAB-mediated suppression of siRNA production was equally efficient whether *tetO* was inserted in the sense or antisense orientations and upstream or downstream of the H1 promoter.

Next, the system was tested for the regulation of truly endogenous genes. p53 and lamin were chosen as targets because highly effective siRNAs directed against these genes were previously identified and well characterized (Brummelkamp *et al.,* 2002; Elbashir *et al.,* 2001). MCF-7 breast cancer cells were used as substrates for p53 downregulation studies (FIG. 6, left). Cells co-transduced with LV-tTR-KRAB and LV-THsi/p53 produced wild-type levels of p53 when cultured in the absence of dox, indicating full repression of siRNA synthesis (lower blot, lane 7). This repression was mediated by tTR-KRAB since p53 was undetectable in cells transduced only with LV-THsi/p53, whether dox was present or not in the culture medium (upper blot, lanes 7 and 8). In contrast, addition of the drug to the dually transduced cells resulted in rates of p53 down modulation as robust as observed in cells containing the constitutively active LV-Hsi/p53 vector (compare lane 8 from lower blot with lanes 5 and 6 from both blots). Similar results were obtained for lamin in HeLa cells transduced with the corresponding siRNA lentivectors (FIG. 6, right). Noteworthy, in both settings the drug-induced production of the siRNAs, hence the suppression of the p53 or lamin target genes, correlated with the expression of the lentivector internal GFP marker, whether examined by Western blot (FIG. 6), FACS or confocal microscopy.

Taken together, these results indicate that the tTR-KRAB-regulated, lentiviral vector-mediated delivery of siRNAs allows for the controllable suppression of cellular genes both with a high degree of efficacy and without significant leakiness. To complete the characterization of this system, its kinetics and dose-responsiveness were defined (FIG. 7). p53 was chosen as a target for these analyses because the half-life of this protein is relatively short, around 12 hrs. In MCF-7 cells dually transduced with the LV-THsi/p53 and LVtTR-KRAB vectors, p53 steady state levels started to decrease as early as 12 hrs after addition of 5 µg/ml dox to the culture medium, and became undetectable by Western blot within 36 hrs (FIG. 7A). This suggests that RNA interference was fully effective in less than 24 hrs, implying that the dox-mediated sequestration of tTR-KRAB rapidly unleashes high rates of siRNA production from the integrated H1 promoters. A dose response analysis further revealed an extreme sensitivity to doxycycline control, while pointing to the possibility of some tuning of the gene suppression. Indeed, whereas p53 downregulation was already apparent at the low dox concentration of 0.004 µg/ml, full-blown suppression was achieved only at a dose of 0.25 µg/ml (FIG. 7B). The anti-p53 siRNA used in this experiment being very efficient, a greater range of dox concentrations may allow for a modulation of the degree of gene knockdown with siRNAs of lower specific activity.

### EXAMPLE 4

### Conditional Gene Knockdown non-human Animals (Ckd)

The present invention relates to the application of the lentivector-mediated and drug-inducible RNA interference for the development of gene knockdown non-human animals. The presented technology exploits the following systems: drug-inducible regulation of polymerase III activity mediated by tetracycline transrepressor (tTR-KRAB); and lentivector mediated transgenesis. The main strategies includes: (1) co-transduction of fertilized oocytes with *tetO*-siRNA and tTR-KRAB lentivectors (*via perivitelline* injection) followed by their transfer into the uterus of foster mothers; (2) co-transduction of fertilized oocytes with *tetO*-siRNA and tTR-KRAB lentivectors (after removal of *zona pellucida*) followed by their maturation into blastocysts *in vitro* and transfer into the uterus of foster mothers; (3) co-transduction of morula or blastocysts with *tetO*-siRNA and tTR-KRAB lentivectors followed by their maturation or/and transfer into uterus of foster mothers; and (4) co-transduction of embryonic stem cells (ES) with *tetO*-siRNA and tTR-KRAB lentivectors followed by their injection into blastocyst and transfer into uterus of foster mothers.

Due to high efficiency and lack of chimerism in first generation approach (1) is preferred for the presented invention. Gene knockdown can be induced at any time of development or adulthood by doxycycline administration.

### EXAMPLE 5

### Generation of Conditional Knockdown Mice Using Transgenic tTR-KRAB Mice

The strategies described in Example 4 may be further applied in the generation of tTR-KRAB transgenic animals, which can then be used for further transgenesis with tet-controllable siRNA vectors.

Therefore, to facilitate generation of cKD mice, transgenic mice expressing tTR-KRAB were generated. The tTR-KRAB mice were generated by transduction of fertilized oocytes (*via perivitelline* injection) with LV-tTR-KRAB-dsRed lentivector followed by their transfer into the uterine ampula of foster mothers. This approach eliminates the need for co-transduction thus the maximizing number of workable phenotypes since fertilized oocytes or blastocys isolated from tTR-KRAB mice can be transduced using above strategies (Example 4) with *tetO*-siRNA lentivector. Moreover, ES cells or any other cell types can be isolated from tTR-KRAB and transduced with *tetO*-siRNA vector to analyze the phenotype after conditional gene down-regulation.

### EXAMPLE 6

### Tissue-Specific Conditional Gene Knockdown non-human Animals

The invention can be also extended to obtain conditional gene knockdown in a tissue-specific manner. This situation can be particularly desirable to analyze knockdown phenotype in a particular cell type or if the gene knockdown in a whole organism is lethal. A stuffer flanked by loxP sites (floxed) is inserted into regulable H1 polymerase III promoter that prevents synthesis of downstream shRNA (FIGS. 8 and 9). Transgenic mice are generated, for example, by transducing fertilized oocytes (*perivitalline* injection; A) that were retrieved from transgenic mice expression Cre recombinase under transcriptional control of tissue the specific promoter, following implantation into foster mothers. Due to the activity of Cre the stuffer will be removed thus activating the H1 promoter and allowing for conditional shRNA synthesis limited to the specific tissue. Conditional Cre (*e.g*. coupled with tamoxifen-inducible nuclear localization signal) can be used in some specific situations. A marker gene can be used as a stuffer to monitor efficiency of tissue-specific excision.

### EXAMPLE 7

### Regulation of a Target Gene in a Tissue-specific Knockdown Mice

The present invention investigates the regulation of a gene in a conditional gene knockdown mice in which the gene knockdown is tissue-specific. Thus, a *tetO* lentivector (*e.g*. LV-TH) carrying cDNA encoding a gene of interest under transcriptional control of a tissue specific promoter will be used to transduce fertilized oocytes obtained from tTR-KRAB mice, for example. Drug administration will be conducted to allow for the regulation of the target gene in a tissue-specific manner in double transgenic mice.

### EXAMPLE 8

### Tissue-specific Conditional Expression of Genes Using tTR-KRAB mice

The invention can be also extended to conditional gene replacement in animals. In that case a mutant form of a targeted gene is introduced into a lentiviral vector (FIG. 10). The mutant form is resistant to RNA interference by insertion of silent mutations into its DNA sequence. The specific modalities of the system allow for the following scenario: in the absence of the drug siRNA synthesis as well as mutant gene expression are repressed by tTR-KRAB. In contrast, presence of the drug leads to knockdown of the wild-type gene expression via RNAi and expression of the mutant allele. Thus, a recessive mutant phenotype can be analyzed in a wild-type background by conditional suppression of the wild-type allele.

### EXAMPLE 9

### Doxycycline-inducible Regulation of exogenous gene expression by tTR-KRAB in vitro and in vivo

This example regards the generation of tTR-KRAB mice, as described elsewhere herein, the generation of conditional transgenic animals, the generation of tissue-specific conditional transgenic animals, and conditional expression *in situ*, such as by regulating expression of exogenous genes.

A skilled artisan recognizes that the methods and compositions described herein are utilized for therapeutic purposes, such as for gene therapy, to inhibit immunorecognition of at least one cell, to treat cancer, and so forth) as well as to provide useful means to study gene function. In particular embodiments, this is achieved through regulation of exogenous gene expression by the system and its respective methods described herein.

The strategies described herein could be greatly facilitated by development of a transgenic mice constitutively expressing tTR-KRAB. The tTR-KRAB mouse could be generated using conventional methods (such as, for example, pronuclear injection or transfection of ES cells) or by lentivector-mediated transgenesis, for example. The tTR-KRAB mouse would serve as a "universal platform" allowing for the conditional expression of genes of interest. The present invention therefore employs a *tetO* lentivector (*e.g*. pLVTH) to deliver a cDNA encoding a gene of interest into tTR-KRAB mice. Expression of this transgene will be governed by the nature of the promoter placed upstream in the vector, and subjected in addition to external agent control (such as at least one drug). Use of tTR-KRAB mice will ensure regulation of the gene of interest in every transduced cell.

In a separate experiment, a *tetO* lentivector (e.g. LV-TH) carrying cDNA encoding a gene of interest will be used to transduce fertilized oocytes obtained from tTR-KRAB mice, followed by their implantation into foster mothers. Drug administration will be conducted to investigate the regulation of the target gene in double transgenic mice.

The conditional expression of the gene of interest could be (a) global (in substantially every cell of the mouse, if the transgene is expressed from a constitutive promoter); (b) tissue-specific (if the transgene is expressed from a tissue-specific promoter); or (c) local (if a vector containing the drug-controllable transgene cassette is administered locally, for instance by injection into a specific organ or region of an organ). For (a) and (b), tTR-KRAB mice can be used as background to generate double transgenic mice (using conventional or lentivector-mediated transgenesis, for example) by delivery of an expression cassette comprising the gene of interest placed downstream of a constitutive or tissue-specific promoter and at least one *tetO* element (the *tetO* element could be placed either upstream or downstream of the expression cassette). One major improvement of the present method over existing techniques is that it allows for the drug-controllable tissue-specific expression of transgenes.

FIG. 11 regards a representative embodiment of drug-controllable transgenesis, wherein a mouse comprising the exemplary TR-KRAB is provided, and a polynucleotide encoding a polynucleotide of interest (illustrated as Gene X, although the polynucleotide may not be a gene per se), such as, for example, one under the control of a ubiquitous promoter (top left of the figure), or one under the control of a tissue-specific promoter (top right of the figure) is introduced to the TR-KRAB mouse for external agent-controllable knockdown of the gene of interest.

### EXAMPLE 10

### Doxycycline-inducible Regulation of cellular gene expression by tTR-KRAB mediated Repression of siRNA production in vitro

This example regards generation of tTR-KRAB cell lines, as described elsewhere herein, and also the generation of conditional siRNA libraries in accordance with the methods and compositions of the present invention.

The present invention can be applied to develop siRNA libraries that would allow high throughput studies, for example, on gene function, drug testing (analysis of drug function in the absence of cellular gene(s), for example), and the like. The strategies described below could be greatly facilitated by development of a cell line or cell liness constitutively expressing tTR-KRAB or an analogous transgene. The tTR-KRAB cell line can be generated using a lentivectoral vector (pLV-tTR-KRAB, for example) or other vectors as described elsewhere herein. The tTR-KRAB cell can serve as a "universal platform" that would allow for conditional expression of cellular genes. The present invention therefore employs a *tetO* lentivector (*e.g*. pLVTH) to deliver siRNA (or siRNA library) to tTR-KRAB cells. Subsequent drug administration is conducted that will allow for downregulation of cellular gene(s). Use of at least one tTR-KRAB cell line will ensure regulation of the gene of interest in substantially every transduced cell. The use of conditional libraries would allow for timed, short-term downregulation of cellular gene(s), thus avoiding potential lethality. Additionally, suppression of potentially lethal effects would allow for amplification and propagation of selected cells for further studies.

Thus, the generation of gene knockdown cell lines is useful, for example, for therapeutic purposes (such as, for example, gene therapy), drug screening, and to study gene function. A safety device for the clinical application of siRNA is also an advantage of this and similar embodiments of the present invention.

In a particular aspect of the invention, the methods and compositions can be applied to develop autologous or allogeneic cell lines for therapeutic purposes. Downregulation of transplantation antigens, for example, (*e.g*. by downregulation of beta2-microglobulin expression *via* RNAi, as an exemplary embodiment) would allow for transplantation of allogeneic cells *(e.g.* the non-limiting examples of islets, hepatocytes, dopaminergic neurons, keratinocytes, *etc.)* while minimizing the risk of rejection by the patient's immune system. The present invention would allow for a switch off of the RNAi in case of adverse effects (*e.g*. uncontrollable replication of the transplanted cells).

### EXAMPLE 11

### Doxycycline-inducible Regulation of cellular gene expression by tTR-KRAB mediated Repression of siRNA production in vivo

This example regards generation of the exemplary tTR-KRAB mice, the generation of conditional knockdown transgenic animals, the generation of tissue-specific conditional knockdown transgenic animals, the generation of conditional siRNA libraries *in vivo,* and the generation ES cell lines carrying tTR-KRAB (ES-tTR-KRAB).

FIG. 11 regards a representative embodiment of drug-controllable knockdown, wherein a mouse comprising the exemplary TR-KRAB is provided, and a polynucleotide encoding a siRNA, such as, for example, one under the control of a ubiquitous Pol III promoter (bottom left of the figure), or one under the control of a tissue-specific Pol III promoter (bottom right of the figure) is introduced to the TR-KRAB mouse for external agent-controllable knockdown.

In certain embodiments, the present invention employs a *tet*O lentivector (*e.g.* pLVTH), for example, to deliver siRNA targeted against a cellular gene of interest to tTR-KRAB mice. Subsequent drug administration will allow for the synthesis of siRNA and downregulation of the cellular gene of interest. Use of tTR-KRAB mice will ensure downregulation of the cellular gene of interest in substantially every transduced cell.

As described for conditional expression of exogenous genes in Example 10, conditional downregulation of cellular genes can be global, tissue-specific, or local. Ability to activate RNAi by drug administration would avoid potentially lethal effects of gene knockdown, thus permitting studies of gene function during late stages of development or adulthood. Moreover, the conditional RNAi would allow for analysis of direct effects of gene knockdown, minimizing possibilities of secondary effects or compensations that may occur during long-term loss of gene function. The present system would be useful in generating animal models mimicking various human genetic defects.

Conditional RNAi would allow for the generation of siRNA libraries *in vivo.* Fertilized oocytes isolated from tTR-KRAB mice can be transduced by siRNA library delivered by the lentivectors (*e.g*. pLVTH). Alternatively, ES-tTR-KRAB cells can be transduced by siRNA library delivered by the lentivectors (*e.g*. pLVTH). The conditional system would prevent potential early lethal effects of RNAi, thus allowing for studying the effect of cellular gene knockdown in development or adulthood. Additionally, suppression of potentially lethal effects of loss of gene function would allow for implantation, amplification and propagation of the mouse libraries (or selected clones) of ES cells for further studies.

### REFERENCES

U.S. Patent 4,683,202
U.S. Patent 4,736,866
U.S. Patent 4,873,191
U.S. Patent 4,873,316
U.S. Patent 5,654,195
U.S. Patent 5,665,577
U.S. Patent 5,894,078
U.S. Patent 5,912,411
U.S. Patent 5,925,565
U.S. Patent 5,928,906
U.S. Patent 5,935,819
U.S. Patent 5,936,138
U.S. Patent 5,981,829
U.S. Patent 5,981,830
U.S. Patent 5,986,171
U.S. Patent 5,994,136
U.S. Patent 5,994,618
U.S. Patent 6,002,066
U.S. Patent 6,008,434
U.S. Patent 6,013,516
U.S. Patent 6,018,097
U.S. Patent 6,018,098
U.S. Patent 6,023,010
U.S. Patent 6,025,539
U.S. Patent 6,136,597
U.S. Patent 6,165,782
U.S. Patent 6,207,455
U.S. Patent 6,340,741
U.S. Patent 6,444,871
U.S. Patent 6,531,123

PCT Appl. WO 00/44914
PCT Appl. WO 01/36646
PCT Appl. WO 01/68836
PCT Appl. WO 99/32619
PCT Appl. WO89/06689
PCT Appl. WO89/06693

European Application Publication No. 264,166

Abremski and Hess, J. Mol. Biol., 259:1509-1514, 1984.
Abremski et al., Cell, 32:1301-1311, 1983.
Akkina, et al., J. Virol., 70:2581-2585, 1996.
Altschul, Proteins 32(1):88-96, 1998.
Altschul, et al., Trends Biochem Sci 23(11):444-7, 1998.
Amin et al., J. Molec. Biol., 214:55-72, 1990.
Anderson, W. F., Science 256:808-813; 1992.
Angel et al., Mol. Cell. Biol., 7:2256, 1987.
Angel et al., Cell, 49:729, 1987b.
Angel et al., Mol. Cell. Biol., 7:2256, 1987a.
Armentano, D. et al., Proc. Natl. Acad. Sci. USA 87:6141-6145, 1990.
Atchison and Perry, Cell, 46:253, 1986.
Atchison and Perry, Cell, 48:121, 1987.
Atchison et al., Cell, 46:253, 1986.
Atchison et al., Cell, 48:121, 1987.
Ayer et al., Mol. Cell. Biol. 16:5772-5781, 1996.
Baichwal and Sugden, In: Gene Transfer, Kucherlapati (Ed.), New York, Plenum Press, 117-148, 1986.
Baim et al., Proc. Natl. Acad. Sci. USA 88:5072-5076, 1991.
Banerji et al., Cell, 27(2 Pt 1):299-308, 1981.
Banerji et al., Cell, 33(3):729-740, 1983.
Banerji et al., Cell, 33(3):729-740, 1983.
Barton and Medzhitov, Proc. Natl. Acad. Sci. USA, 99:14943-14945, 2002.
Bellefroid et al., Proc. Natl. Acad. Sci. USA, 88:3608-3612, 1991.
Berkhout et al., Cell, 59:273-282, 1989.
Blanar et al., EMBO J., 8:1139, 1989.
Blomer et al., J. Virol., 71:6641-6649, 1997.
Bodine and Ley, EMBO J., 6:2997, 1987.
Boshart et al., Cell, 41:521, 1985.
Bosze et al., EMBO J., 5(7):1615-1623, 1986.
Braddock et al., Cell, 58:269, 1989.
Bradley et al., Nature, 309:255-258, 1984.
Brinster, et al., Proc. Natl. Acad. Sci. USA, 82:4438-4442, 1985.
Brown et al., Cell 49:603-612, 1987.
Brummelkamp et al. Science, 296:550-553, 2002.
Bulla and Siddiqui, J. Virol., 62:1437, 1986.
Byrne and Ruddle, Proc. Natl. Acad. Sci. USA 86:5473-5477, 1989.
Calame and Eaton, Adv. Immunol. 43:235-275, 1988.
Campbell and Villarreal, Mol. Cell. Biol., 8:1993, 1988.
Campere and Tilghman, Genes and Dev., 3:537, 1989.
Campes and Tilghman, Genes Dev. 3:537-546, 1989.
Campo et al., Nature, 303:77, 1983.
Carbonelli et al., FEMS Microbiol. Lett., 177(1):75-82, 1999.
Carmell et al., Nat. Struct. Biol. 10(2):91-92, 2003.
Celander and Haseltine, J. Virology, 61:269, 1987.
Celander et al., J. Virology, 62:1314, 1988.
Chandler et al., Cell, 33:489, 1983.
Chandler et al., Proc. Natl. Acad. Sci. USA, 94(8):3596-601, 1997.
Chang et al., Mol. Cell. Biol., 9:2153, 1989.
Charneau et al., Mol. Biol. 241:651-662, 1994.
Chatterjee et al., Proc. Natl. Acad. Sci. USA, 86:9114, 1989.
Choi et al., Cell, 53:519, 1988.
Chowdhury, J. R. et al., Science 254:1802-1805, 1991.
Cocea, Biotechniques, 23(5):814-816,1997.
Cohen et al., J. Cell. Physiol., 5:75, 1987.
Corbeau, et al., Proc. Nat. Acad. Sci. USA, 93:14070-14075, 1996.
Costa et al., Mol. Cell. Biol., 8:81, 1988.
Coupar et al., Gene, 68:1-10, 1988.
Cournoyer, D., et al., Curr. Opin. Biotech. 1:196-208, 1990.
Craig, Ann. Rev. Genet., 22:77-105, 1988.
Cregg and Madden, Mol. Gen. Genet., 219:320-323, 1989.
Cripe et al., EMBO J., 6:3745, 1987.
Cristiano, R. J. et al., Proc. Natl. Acad Sci. USA 90:2122-2126, 1993.
Culotta and Hamer, Mol. Cell. Biol., 9:1376, 1989.
Cultraro et al., Mol Cell. Biol. 17:2353-2359, 1997.
Dai, Y. et al., Proc. Natl. Acad. Sci. USA 89:10892-10895, 1992.
Dandolo et al., J. Virology, 47:55-64, 1983.
de Villartay, Nature, 335:170-174, 1988.
De Villiers et al., Nature, 312(5991):242-246, 1984.
Deschamps et al., Science, 230:1174-1177, 1985.
Deuschle et al., Proc. Natl. Acad. Sci. USA 86:5400-5405, 1989.
Deuschle et al., Science 248:480-483, 1990.
Deuschle et al., Mol. Cell. Biol., 15:1907-1914, 1995.
Devereux, et al., Nucleic Acids Res., 12(1):387. 1984.
Devroe and Silver, BMCBiotechnol., 2(1):15, 2002.
Donello et al., J. Virol., 72:5085-5092, 1998.
Dull et al., J. Virol., 72:8463-8471, 1998.
Echols, J. Biol. Chem., 265:14697-14700, 1990.
Edbrooke et al., Mol. Cell. Biol., 9:1908, 1989.
Edlund et al., Science, 230:912-916, 1985.
Elbashir et al., Nature, 411:494-498, 2001.
Epstein et al., Mol. Cell. Biol. 18:4118-4130,1998.
Evans et al., Nature, 292:154-156, 1981.
Feng and Holland, Nature, 334:6178, 1988.
Ferry, N. et al., Proc. Natl. Acad. Sci. USA 88:8377-8381, 1991.
Figge et al., Cell 52:713-722, 1988.
Firak and Subramanian, Mol. Cell. Biol., 6:3667, 1986.
Flanagan and Fennewald, J. Molec. Biol., 206:295-304, 1989.
Foecking and Hofstetter, Gene, 45(1):101-105, 1986.
Friedman et al., Genes Dev. 10:2067-2078, 1996.
Friedmann, Science, 244:1275-1281,1989.
Fuerst et al., Proc. Natl. Acad. Sci. USA 86:2549-2553, 1989.
Fussenegger et al., Nat. Biotech., 18:1203-1208, 2000.
Fujita et al., Cell, 49:357, 1987.
Gilles et al., Cell, 33:717, 1983.
Gilles et al., Cell, 33:717, 1983.
Ginsberg et al., Cancer Res. 15:3542-3546, 1998.
Glasgow et al., J. Biol. Chem., 264:10072-10082, 1989.
Gloss et al., EMBO J., 6:3735, 1987.
Godbout et al., Mol. Cell. Biol., 8:1169, 1988.
Golic and Lindquist, Cell, 59:499-509, 1989.
Goodbourn and Maniatis, Proc. Natl. Acad. Sci. USA, 85:1447, 1988.
Goodbourn et al., Cell, 45:601, 1986.
Gossen and Bujard, Proc. Natl. Acad. Sci. USA, 89:5547-5551, 1992.
Gossen et al., Science, 268:1766-1769, 1995.
Gossler, et al., Proc. Natl. Acad. Sci. USA, 83, 9065-9069, 1986.
Gralla et al., Proc. Natl. Acad. Sci. USA 88: 8558-62, 1991.
Greene et al., Immunology Today, 10:272, 1989
Grossched1 and Baltimore, Cell, 41:885, 1985.
Grosschedl et al., Cell, 41:885, 1985.
Grosveld et al., Cell 51:975-985, 1987.
Gupta et al., Oncogene 16:1149-1159, 1998.
Gussen et al., Proc. Natl. Acad. Sci. USA, 89:5547-5551, 1992.
Haffter and Bickle, EMBO J., 7:3991-3996, 1988.
Haslinger and Karin, Proc. Natl. Acad. Sci. USA, 82:8572, 1985.
Hauber and Cullen, J. Virology, 62:673, 1988.
Hen et al., Nature, 321:249, 1986.
Hennighausen et al., J. Cell. Biochem., 59:463472, 1995.
Hensel et al., Lymphokine Res., 8:347, 1989.
Hermonat and Muzycska, Proc. Natl. Acad. Sci. USA, 81:6466-6470, 1984.
Herr and Clarke, Cell, 45:461, 1986.
Herz, J. and Gerard, R. D., Proc. Natl. Acad Sci. USA 90:2812-2816, 1993.
Higgins et al., Computer Applis. Biosci., (CABIOS), 8(2):189-191, 1992.
Hillen, W. and Schollmeier, K. Nucl. Acids Res. 11:525-539, 1983
Hirochika et al., J. Virol., 61:2599, 1987.
Hirsch et al., Mol. Cell. Biol., 10:1959, 1990.
Hoess and Abremski, Proc. Natl. Acad. Sci. USA, 81:1026-1029, 1984.
Hogan et al., In: Manipulating the Mouse Embryo, A Laboratory Manual, 2Ed., Cold Spring Harbor Laboratory Press, 1994.
Hogan et al., J Embryol Exp Morphol 97:95-110, 1986.
Holbrook et al., Virology, 157:211, 1987.
Horlick and Benfield, Mol. Cell. Biol., 9:2396, 1989.
Horwich et al. J. Virol., 64:642-650, 1990.
Hottiger et al., Yeast 10: 283-96, 1994.
Hu and Davidson, Cell 48:555-566, 1987.
Huang et al., Cell, 27:245, 1981.
Hubner et al., J. Molec. Biol., 205:493-500, 1989.
Hug et al., Mol. Cell. Biol., 8:3065, 1988.
Hunger-Bertling et al., Mol. Cell. Biochem., 92:107-116, 1990.
Hutvagner et al., Science, 293:834-838, 2001.
Hwang et al., Mol. Cell. Biol., 10:585, 1990.
Hwu, P. et al., J. Immunol. 150:4104-4115, 1993.
Imagawa et al., Cell, 51:251, 1987.
Imbra and Karin, Nature, 323:555, 1986.
Imler et al., Mol. Cell. Biol., 7:2558, 1987.
Imler et al., Mol. Cell. Biol., 7:2558, 1987.
Imperiale and Nevins, Mol. Cell. Biol., 4:875, 1984.
Jacque et al., Nature, 418:435-438, 2002..
Jaenich, Proc. Natl. Acad. Sci. USA, 73:1260-1264, 1976.
Jaenisch, Science, 240:1468-1474, 1988.
Jahner et al., Nature, 298:623-628, 1982.
Jahner, et al., Proc. Natl. Acad. Sci. USA, 82:6927-6931, 1985.
Jakobovits et al., Mol. Cell. Biol., 8:2555, 1988.
Jameel and Siddiqui, Mol. Cell. Biol., 6:710, 1986.
Jaynes et al., Mol. Cell. Biol., 8:62, 1988.
Johnson et al., Mol. Cell. Biol., 9:3393, 1989.
Johnston, Microbiol Rev 51: 458-76, 1987.
Kadesch and Berg, Mol. Cell. Biol., 6:2593, 1986.
Kafri et al., Nat. Genet., 17:314-317, 1997.
Karin et al., Mol. Cell. Biol., 7:606, 1987.
Karin et al., Mol. Cell. Biol., 7:606, 1987.
Katinka et al., Cell, 20:393, 1980.
Kawamoto et al., Mol. Cell. Biol., 8:267, 1988.
Kay, M. A. et al., Human Gene Therapy 3:641-647, 1992.
Kessel and Cruss, Science 249:374-379, 1990.
Kiledjian et al., Mol. Cell. Biol., 8:145, 1988.
Kiledjian et al., Mol. Cell. Biol., 8:145, 1988.
Kim et al., J. Virol., 69:2565-2573, 1995.
Kistner et al., Proc. Natl. Acad. Sci. USA, 93:10933-10938, 1996.
Klamut et al., Mol. Cell. Biol., 10:193,1990.
Klimatcheva et al., Frontiers in Bioscience, 4:481-496, 1999.
Koch et al., Mol. Cell. Biol., 9:303, 1989.
Kramer, et al., Biotechnol. Bioengin., 83(7):810-820, 2003.
Kriegler and Botchan, In: Eukaryotic Viral Vectors, Gluzman (Ed.), Cold Spring Harbor: Cold Spring Harbor Laboratory, NY, 1982.
Kriegler and Botchan, Mol. Cell. Biol., 3:325, 1983.
Kriegler et al., Cell, 38:483, 1984.
Kriegler et al., Cell, 53:45, 1988.
Kuhl et al., Cell, 50:1057, 1987.
Kunz et al., Nucl. Acids Res., 17:1121, 1989.
Labow et al., Mol. Cell. Biol. 10:3343-3356, 1990.
Laherty et al., Cell 89:349-356, 1997.
Lapinskas et al., Curr Genet 24: 388-93, 1993.
Larsen et al., Proc Natl. Acad. Sci. USA., 83:8283, 1986.
Larsson et al., Oncogene 15:737-748, 1997.
Laspia et al., Cell, 59:283, 1989.
Latimer et al., Mol. Cell. Biol., 10:760, 1990.
Lee et al., Nature, 294:228, 1981.
Lee et al., Nucleic Acids Res., 12:4191-206, 1984.
Lesk, In; Computational Molecular Biology, Oxford University Press, NY, 1988.
Levinson et al., Nature, 295:79, 1982.
Lin et al., Mol. Cell. Biol., 10:850, 1990.
Liu et al., Cancer Gene Ther. 5:3-28, 1998.
Lois et al., Science, 295:868-872, 2002.
Luria et al., EMBO J., 6:3307, 1987.
Luria et al., EMBO J., 6:3307, 1987.
Lusky and Botchan, Proc. Natl. Acad. Sci. USA, 83:3609, 1986.
Lusky et al., Mol. Cell. Biol., 3:1108, 1983.
Macejak and Sarnow, Nature, 353:90-94, 1991.
Magari et al., J. Clin. Invest., 100:2865-2872, 1997.
Majors and Varmus, Proc. Natl. Acad. Sci. USA, 80:5866,1983.
Malynn et al., Cell, 54:453-460, 1988.
Margolin et al., Proc. Natl. Acad. Sci. USA, 91:4509-4513, 1994.
Markowitz, et al., J. Virol., 62:1120-1124, 1988.
Matsuzaki et al., J. Bacteriol., 172:610-618, 1990.
McNeall et al., Gene, 76:81, 1989.
McKnight et al., Cell 37:253-262, 1984.
Mercier et al., J. Bacteriol., 172:3745-757, 1990.
Mikkola et al., Nature, 421:547-551, 2003.
Miksicek et al., Cell, 46:203, 1986.
Miller, A. D., Nature 357:455-460, 1992.
Miyoshi et al., Science, 283:682-686, 1999:
Montgomery et al., Proc. Natl. Acad. Sci. USA, 95:15502-15507, 1998.
Moosmann et al., Biol. Chem., 378:669-677, 1997.
Mordacq and Linzer, Genes and Dev., 3:760, 1989.
Moreau et al., Nucl. Acids Res., 9:6047, 1981.
Muesing et al., Cell, 48:691, 1987.
Naldini et al., Proc. Natl. Acad. Sci. USA, 93:11382-11388, 1996b.
Naldini et al.., Science, 272:263-267, 1996a.
Ng et al., Nuc. Acids Res., 17:601, 1989.
Ng et al., Mol. Cell. Biol. 5:2720-2732, 1985.
No et al., Proc. Natl. Acad. Sci. USA, 93:3346-3351, 1996.
Oligino et al., Gene Ther. 5: 491-6, 1998.
Ondek et al., EMBO J., 6:1017, 1987.
Ornitz et al., Mol. Cell. Biol., 7:3466, 1987.
Page, et al., J. Virol., 64:5270-5276, 1990.
Palmiter et al., Nature, 300:611, 1982.
Parsons et al., J. Biol. Chem., 265:4527-4533, 1990.
Pech et al., Mol. Cell. Biol., 9:396, 1989.
Pelletier and Sonenberg, Nature, 334:320-325, 1988.
Pengue et al., Nucl. Acids Res. 22:2908-2914, 1994.
Perez-Stable and Constantini, Mol. Cell. Biol., 10:1116, 1990.
Pfeifer et al., Proc. Natl. Acad Sci. USA, 99:2140-2145, 2002..
Picard and Schaffner, Nature, 307:83, 1984.
Picard et al., Nature, 307:83, 1984.
Pinkert et al., Genes and Dev., 1:268, 1987.
Ponta et al., Proc. Natl. Acad. Sci. USA, 82:1020, 1985.
Porton et al., Mol. Cell. Biol., 10:1076, 1990.
Porton et al., Mol. Cell. Biol., 10:1076, 1990.
Poyart-Salmeron et al., EMBO J., 8:2425-2433, 1989.
Qin et al., Proc. Natl. Acad. Sci. USA, 100:183-188,2003.
Quantin, B. et al., Proc. Natl. Acad. Sci. USA 89:2581-2584, 1992.
Queen and Baltimore, Cell, 35:741, 1983.
Queen et al,. Cell, 35:741, 1983.
Queva et al., Oncogene 16:967-977, 1998.
Quinn et al., Mol. Cell. Biol., 9:4713, 1989.
Redondo et al., Science, 247:1225, 1990.
Redondo et al., Science, 247:1225, 1990.
Reisman and Rotter, Mol. Cell. Biol., 9:3571, 1989.
Resendez Jr. et al., Mol. Cell. Biol., 8:4579, 1988.
Ridgeway, In: Vectors: A survey of molecular cloning vectors and their uses, Rodriguez and Denhardt (Eds.), Stoneham: Butterworth, 467-492, 1988.
Ripe et al., Mol. Cell. Biol., 9:2224, 1989.
Rittling et al., Nuc. Acids Res., 17:1619, 1989.
Robertson et al., Nature, 322:445-448, 1986.
Rosen et al., Cell, 41:813, 1988.
Rosenfeld, M. A. et al., Cell 68:143-155, 1992.
Rubinson et al., Nat. Genet., 33:401-406, 2003.
Ruzzi et al., Mol Cell Biol 7: 991-7, 1987.
Sakai et al., Genes and Dev., 2:1144, 1988.
Sambrook et al., In: Molecular cloning, Cold Spring Harbor Laboratory Press, Cold Spring Harbor, NY, 1989.
Satake et al., J. Virology, 62:970, 1988.
Sato et al., J. Bacteriol., 172:1092-1098, 1990.
Schaffner et al., J. Mol. Biol., 201:81, 1988.
Schwartz and Sadowski, J. Molec. Biol., 205:647-658, 1989.
Searle et al., Mol. Cell. Biol., 5:1480, 1985.
Senatore et al., Gene, 234:381-394, 1999.
Sgouras et al., EMBO J. 14:4781-4793, 1995.
Sharp and Marciniak, Cell, 59:229, 1989.
Sharp, Genes Dev. 15:485-490, 2001.
Shaul and Ben-Levy, EMBO J., 6:1913, 1987*.*
Sherman et al., Mol. Cell. Biol., 9:50, 1989.
Shimada et al., J. Clin. Investig., 88:1043-1047, 1991.
Shinagawa and Ishii, Genes Devel., 17:1340-1345, 2003.
Sleigh and Lockett, J. EMBO, 4:3831,1985.
Smith, In: Biocomputing: Informatics and Genome Projects, Academic Press, New York, 1993, 1986.
Sommer et al., J. Biol. Chem. 273:6632-6642, 1998.
Spalholz et al., Cell, 42:183, 1985.
Spandau and Lee, J. Virology, 62:427,1988.
Spandidos and Wilkie, EMBO J., 2:1193, 1983.
Spencer et al., Science, 262:1019-1024, 1993.
Stark et al., Cell, 58:779-790, 1989.
Stephens and Hentschel, Biochem. J., 248:1, 1987.
Sternberg et al.. Cold Spring Harbor Symp. Quant. Biol.. 45:297-309, 1981.
Stewart et al., EMBO J., 6:383-388, 1987.
Strain and Culotta, Mol Gen Genet 251: 139-45, 1996.
Stuart et al., Nature, 317:828, 1985.
Stuber, D. and Bujard, H., Proc. Natl. Acad. Sci. USA 78:167-171, 1981.
Sui et al. Proc. Natl. Acad. Sci. USA, 99:5515-5520, 2002.
Sullivan and Peterlin, Mol. Cell. Biol., 7:3315, 1987.
Sullivan et al., Mol. Cell. Biol., 7:3315, 1987.
Swartzendruber and Lehman, J. Cell. Physiology, 85:179, 1975.
Takebe et al., Mol. Cell. Biol., 8:466, 1988.
Tavernier et al., Nature, 301 : 634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:15160, 1989.
Thiesen et al., J. Virology, 62:614, 1988.
Thiesen et al., New Biologist 2:363-374, 1990.
Thompson et al., Nucleic Acids Res., 22:4673-4680, 1994.
Tiscornia et al., Proc. Natl. Acad. Sci. USA, 100:1844-1848, 2003.
Tovar, K. et al., Mol. Gen. Genet. 215:76-80, 1988.
Treisman, Cell, 42:889, 1985*.*
Tronche et al., Mol. Biol. Med., 7:173, 1990.
Trudel and Constantini, Genes and Dev. 6:954, 1987.
Tyndell et al., Nuc. Acids. Res., 9:6231, 1981.
Unger, B. et al., Nucl Acids Res. 12:7693-7703, 1984.
van Beusechem, V. W. et al., Proc. Natl. Acad Sci. USA 89:7640-7644, 1992.
Van der Putten et al., Proc. Natl. Acad. Sci. USA, 82:6148-6152, 1985.
Vannice and Levinson, J. Virology, 62:1305, 1988.
Vasseur et al., Proc Natl. Acad. Sci. U.S.A., 77:1068, 1980.
Wang and Calame, Cell, 47:241, 1986.
Wang et al., Nature Biotech., 15:239-243, 1997.
Wang, Proc. Natl. Acad. Sci. USA, 93:8180-8184, 1994.
Waterhouse et al., Trends Plant Sci., 6(7):297-301, 2001.
Waters, S. H. et al., Nucl. Acids Res. 11:6089-6105, 1983.
Weber et al., Cell, 36:983, 1984.
Weber et al., Nucl. Acids Res., 31:e71, 2003b.
Weber et al., Nucl. Acids Res., 31,:e69, 2003a.
Weber et al., Nat. Biotech., 20:901-907, 2002.
Weinberger et al. Mol. Cell. Biol., 8:988, 1984.
Weinberger et al., Mol. Cell. Biol., 8:988, 1984.
Weisberg et al., In: Lambda II, Hendrix et al. (Eds.), Cold Spring Harbor Press, Cold Spring Harbor, NY, 211-250, 1983.
Wilson, J. M et al., Proc. Natl. Acad. Sci. USA 85:3014-3018, 1988.
Wilson, J. M. et al., J. Biol. Chem. 267:963-967, 1992.
Winoto and Baltimore, Cell 59:649, 1989.
Winoto et al., Cell, 59:649, 1989.
Witzgall et al., Proc. Nat'l Acad. Sci. USA 91:4514-4518, 1994.
Wolff, J. A. et al., Science 247:1465-1468, 1990.
Xia et al. Nature Biotech., 20:1006-1010, 2002.
Yan et al., Proc. Nat' Acad. Sci. USA 95:8298-8303, 1998.
Yutzey et al. Mol. Cell. Biol., 9:1397, 1989.
Zamore et al., Cell, 101:25-33, 2000.
Zennou et al., Cell, 101:173-185, 2000.
Zufferey et al., J. Virol., 72:9873-9880, 1998.
Zufferey et al., J. Virol., 73:2886-2892, 1999.
Zufferey et al., Nat. Biotechnol., 15:871-875, 1997.

## Claims

1. A system for controlling gene expression comprising:
(a) a polynucleotide construct comprising a regulatable Pol I or Pol II or Pol III promoter comprising a tetracycline operator (*tetO*) polynucleotide sequence operably linked to at least one polynucleotide encoding siRNAs;
(b) a polynucleotide encoding a drug-controllable repressor fusion protein that comprises the DNA binding domain of the tetracycline repressor (tTR) fused to the KRAB repression domain of human Kox-1 (tTR-KRAB); and
wherein the constructs of (a) and (b) are on one or more vectors

2. The system of claim 1, wherein the vector or vectors is a lentiviral vector, an MLV vector, an AAV vector, a plasmid vector or an adenoviral (Adv or Ad) vector.

3. The system of claim 1, wherein the regulatable promoter comprising a tetracycline operator (*tetO*) polynucleotide sequence operably linked to the polynucleotide sequence encoding the siRNA and the polynucleotide sequence encoding the siRNA are comprised in the U3 region of the 3' long terminal repeat of a lentiviral vector.

4. The system of claim 1, wherein the polynucleotide encoding the fusion protein is comprised within a second, separate vector from the vector comprising the constructs of (a).

5. The system of claim 4, wherein the second vector comprising the polynucleotide encoding the fusion protein is a lentiviral vector, a MLV vector, an AAV vector, a plasmid vector or an adenoviral (Adv or Ad) vector.

6. The system of claim 1, wherein the polynucleotide encodes siRNA that forms a stem-and-loop structure, or a hairpin.

7. The system of claim 1, further comprising a compound that may be administered to the cell that controls the binding of the fusion protein to the *tetO* polynucleotide sequence.

8. The system of claim 7, wherein the compound is a tetracycline.

9. The system of claim 8, wherein the tetracycline is doxycycline.

10. The system of claim 1, wherein the polynucleotide encoding siRNAs is further defined as polynucleotides encoding an siRNA library.

11. A transgenic cell comprising the constructs (a) and (b) of claim 1.

12. The transgenic cell of claim 11, further defined as a non-human oocyte.

13. A transgenic, non-human animal comprising the constructs (a) and (b) of claim 1 or cells in accordance with claim 11 or 12.

## Patentansprüche

1. System zur Kontrolle von Genexpression, das Folgendes umfasst:
(a) ein Polynukleotid-Konstrukt, das einen regulierbaren Pol I oder Pol II oder Pol III Promotor umfasst, der eine Tetracyclin-Operator (tetO)-Polynukleotidsequenz umfasst, die mit mindestens einem Polynukleotid, das siRNAs kodiert, funktionell verbunden ist;
(b) ein Polynukleotid, das ein durch einen Wirkstoff kontrollierbares Repressor-Fusionsprotein kodiert, das die DNA-Bindungsdomäne des Tetracyclin-Repressors (tTR) umfasst, der an die KRAB-Repressionsdomäne von humanem Kox-1 fusioniert ist (tTR-KRAB); und
worin die Konstrukte von (a) und (b) auf einem oder mehreren Vektoren liegen.

2. System nach Anspruch 1, worin der Vektor oder die Vektoren ein lentiviraler Vektor, ein MLV-Vektor, ein AAV-Vektor, ein Plasmidvektor oder ein adenoviraler (Adv- oder Ad-) Vektor ist/sind.

3. System nach Anspruch 1, worin der regulierbare Promotor, der eine Tetracyclin-Operator (*tetO*)-Polynukleotidsequenz umfasst, die mit der Polynukleotidsequenz, die die siRNA kodiert, funktionell verbunden ist, und die Polynukleotidsequenz, die die siRNA kodiert, in der U3-Region der 3' langen terminalen Sequenzwiederholung eines lentiviralen Vektors enthalten sind.

4. System nach Anspruch 1, worin das Polynukleotid, das das Fusionsprotein kodiert, innerhalb eines zweiten, vom Vektor, der die Konstrukte von (a) umfasst, separaten Vektors enthalten ist.

5. System nach Anspruch 4, worin der zweite Vektor, der das Polynukleotid umfasst, das das Fusionsprotein kodiert, ein lentiviraler Vektor, ein MLV-Vektor, ein AAV-Vektor, ein Plasmidvektor oder ein adenoviraler (Adv- oder Ad-) Vektor ist.

6. System nach Anspruch 1, worin das Polynukleotid siRNA kodiert, die eine Stamm-Schleifen-Struktur oder eine Haarnadel bildet.

7. System nach Anspruch 1, das weiter eine Verbindung umfasst, die der Zelle verabreicht werden kann, die die Bindung des Fusionsproteins an die *tetO-*Polynukleotidsequenz kontrolliert.

8. System nach Anspruch 7, worin die Verbindung ein Tetracyclin ist.

9. System nach Anspruch 8, worin das Tetracyclin Doxycyclin ist.

10. System nach Anspruch 1, worin das Polynukleotid, das siRNAs kodiert, weiter als Polynukleotide, die eine siRNA-Bibliothek kodieren, definiert ist.

11. Transgene Zelle, die die Konstrukte (a) und (b) von Anspruch 1 umfasst.

12. Transgene Zelle nach Anspruch 11, die weiter als eine nicht-humane Oozyte definiert ist.

13. Transgenes, nicht-humanes Tier, das die Konstrukte (a) und (b) von Anspruch 1 oder Zellen nach Anspruch 11 oder 12 umfasst.

## Revendications

1. Système pour contrôler l'expression génétique comprenant :
(a) une construction polynucléotidique comprenant un promoteur Pol I ou Pol II ou Pol III pouvant être régulé comprenant une séquence polynucléotidique d'opérateur de tétracyclines *(tetO)* liée de manière opérationnelle à au moins un polynucléotide codant des siARN ;
(b) un polynucléotide codant une protéine de fusion répresseur contrôlable avec des médicaments qui comprend le domaine de liaison ADN du répresseur de tétracyclines (tTR) collé au domaine de répression KRAB de Kox-1 (tTR-KRAB) humain ; et
dans lequel les constructions de (a) et (b) sont sur un ou plusieurs vecteurs.

2. Système selon la revendication 1, dans lequel le vecteur ou les vecteurs est/sont un vecteur lentiviral, un vecteur MuLV, un vecteur VAA, un vecteur du plasmide ou un vecteur adénoviral (adv ou Ad).

3. Système selon la revendication 1, dans lequel le promoteur pouvant être régulé comprenant une séquence polynucléotidique d'opérateur de tétracyclines (*tetO*) liée de manière opérationnelle à la séquence polynucléotidique codant le siARN et la séquence polynucléotidique codant le siARN sont compris dans la région U3 de la répétition longue terminale 3' d'un vecteur lentiviral.

4. Système selon la revendication 1, dans lequel le polynucléotide codant la protéine de fusion est compris dans un deuxième vecteur séparé du vecteur comprenant les constructions de (a).

5. Système selon la revendication 4, dans lequel le deuxième vecteur comprenant le polynucléotide codant la protéine de fusion est un vecteur lentiviral, un vecteur MuLV, un vecteur VAA, un vecteur du plasmide ou un vecteur adénoviral (adv ou Ad).

6. Système selon la revendication 1, dans lequel le polynucléotide code le siARN qui forme une structure tige-boucle, ou une épingle.

7. Système selon la revendication 1, comprenant en outre un composé qui peut être administré à la cellule qui contrôle la liaison de la protéine de fusion à la séquence polynucléotidique *tetO*.

8. Système selon la revendication 7, dans lequel le composé est une tétracycline.

9. Système selon la revendication 8, dans lequel la tétracycline est la doxycycline.

10. Système selon la revendication 1, dans lequel le polynucléotide codant les siARN est en outre défini comme polynucléotides codant une bibliothèque de siARN.

11. Cellule transgénique comprenant les constructions (a) et (b) selon la revendication 1.

12. Cellule transgénique selon la revendication 11, en outre définie comme un ovocyte non humain.

13. Animal non humain transgénique comprenant les constructions (a) et (b) selon la revendication 1 ou des cellules selon la revendication 11 ou la revendication 12.
